# EUROPEAN PATENT APPLICATION

(11) **EP 3 449 922 A1**
(43) Date of publication of application: **06.03.2019**
(21) Application number: 17788869.0
(22) Date of filing: 27.04.2017
(51) Int. Cl.: A61K 31/416, A61P 35/00

(54) **INDAZOLE DERIVATIVES FOR CANCER TREATMENT**

(30) Priority: 27.04.2016 ES 201630539
(71) Applicant: Servicio Andaluz de Salud, 41001 Sevilla (ES); Agencia Estalal Consejo Superior de Investigaciones Cientificas, 28006 Madrid (ES)
(72) Inventor: PÉREZ SIMÓN, José Antonio, 41071 Sevilla (ES); BARBADO GONZÁLEZ, Maria Victoria, 41013 Sevilla (ES); MEDRANO DOMÍNGUEZ, Maite, 41013 Sevilla (ES); CAMPILLO MARTÍN, Nuria Eugenia, 28006 Madrid (ES); PÁEZ PROSPER, Juan Antonio, 28006 Madrid (ES); GONZÁLEZ NARANJO, Pedro José, 28006 Madrid (ES)
(74) Representative: Hoffmann Eitle
(86) International application number: PCT/ES2017/070261
(87) International publication number: WO 2017/186999

(57) **Abstract**

The invention relates to the use of a series of indazole compounds and the derivatives thereof in the production of a medicinal product to be administered alone or in combination with another suitable active ingredient in the treatment of cancer, and more specifically the treatment of hematological cancer.

## Description

### FIELD OF THE INVENTION

The present invention is comprised in the field of medicine and pharmacy, and relates to the use of new compounds and the pharmaceutically acceptable derivatives thereof in the production of a medicinal product for the prevention, relief, improvement, and/or treatment of cancer, more specifically for the treatment of hematological cancer, and even more preferably for the treatment of acute myeloid leukemia (AML) and monoclonal gammopathies generally, and multiple myeloma (MM) particularly.

### BACKGROUND OF THE INVENTION

Hematological cancer, also known as hematological neoplasms or hematological malignancies, are a heterogeneous group of malignant diseases affecting the blood, bone marrow, and lymph nodes.

The WHO classifies hematological neoplasms according to their myeloid or lymphoid origin (Vardiman and James W, 2009. Blood Journal 114:937-951).

### Classification of MYELOID hematological neoplasms

### Chronic myeloproliferative neoplasms (CMN)

BCR-ABL positive chronic myeloid leukemia
Chronic neutrophilic leukemia
Polycythemia vera
Primary myelofibrosis
Essential thrombocytopenia
Chronic eosinophilic leukemia
Systemic mastocytosis
Unclassifiable myeloproliferative neoplasms

### Myeloid and lymphoid neoplasms with eosinophilia and PDGFRA, PDGFRB, or FGFR1 abnormalities

### Myelodysplastic syndromes (MDS)

Refractory cytopenia with unilineage dysplasia
Refractory sideroblastic anemia
Refractory cytopenia with multilineage dysplasia
Refractory anemia with excess blasts
Myelodysplastic syndrome with isolated del(5q)
Unclassificable myelodysplastic syndrome
Childhood myelodysplastic syndrome

### Myelodysplastic/myeloproliferative neoplasms (MDS/MPN)

Chronic myelomonocytic leukemia
BCR-ABL negative chronic atypical myeloid leukemia
Juvenile myelomonocytic leukemia
Unclassificable myelodysplastic/myeloproliferative neoplasm

### Acute myeloid leukemias (AML)

AML with recurrent genetic abnormalities
   AML with t(8;21)(q22;q22); RUNX1
AML with myelodysplasia-related changes
Prior therapy-related AML
AML without characteristics typical of the preceding categories
Myeloid sarcoma
Down syndrome-related myeloid proliferations
Blastic plasmocytoid dendritic cell neoplasm

### Acute leukemias of ambiguous lineage

### Classification of LYMPHOID hematological neoplasms

### Precursor cell lymphoid neoplasms

B-cell lymphoblastic lymphoma/leukemia
T-cell lymphoblastic lymphoma/leukemia

### Mature B-cell neoplasms

Chronic lymphatic leukemia/lymphocytic lymphoma
B-cell prolymphocytic leukemia
Splenic marginal zone B-cell lymphoma
Tricoleukemia
Lymphoplasmocytic lymphoma/Waldenström's macroglobulinemia
Plasma cell myeloma/extramedullary plasmacytoma
Extranodal marginal zone lymphoma of mucosa-associated lymphoid tissue (MALT lymphoma)
Nodal marginal zone lymphoma
Follicular lymphoma
Primary cutaneous centrofollicular lymphoma
Mantle cells lymphoma
Diffuse large B-Cell lymphoma, unspecified
T-cell and histiocyte-rich large B-cell lymphoma
Primary large B-cell lymphoma of the central nervous system
Primary cutaneous large B-cell lymphoma, leg type
Lymphomatoid granulomatosis
Primary mediastinal large B-cell lymphoma
Intravascular lymphoma
Plasmablastic lymphoma
Primary lymphoma of cavities
Burkitt's lymphoma

### Mature NK-cell and T-cell neoplasms

T-cell prolymphocytic leukemia
Granular T-cell lymphocytic leukemia
Granular NK-cell lymphoproliferative process, indolent
Aggressive NK-cell leukemia
Adult T-cell lymphoma/leukemia
Nasal type extranodal NK/T-cell lymphoma
Enteropathy-associated T-cell lymphoma
Hepatosplenic lymphoma
Subcutaneous panniculitis-like T-cell lymphoma
Mycosis fungoides/Sézary syndrome
Primary cutaneous CD30-positive lymphoproliferative disorders
Lymphomatoid papulosis
Primary cutaneous anaplastic large cell lymphoma
Primary cutaneous Tg-d-cell lymphoma
Peripheral T-cell lymphoma, unspecified
Angioimmunoblastic T-cell lymphoma
Anaplastic large cell lymphoma, ALK+
Anaplastic large cell lymphoma, ALK-

### Hodgkin's lymphoma

Nodular lymphocyte-predominant Hodgkin's lymphoma
Classic Hodgkin's lymphoma
   Classic nodular sclerosis Hodgkin's lymphoma
   Classic lymphocyte-rich Hodgkin's lymphoma
   Classic mixed cellularity Hodgkin's lymphoma
   Classic lymphocyte-depleted Hodgkin's lymphoma

Acute myeloid leukemia (AML, or LMA in Spanish) is known by many other names, including acute myelocytic leukemia, acute myelogenous leukemia, acute granulocytic leukemia, and acute non-lymphocytic leukemia. It is the most common type of acute leukemia in adults. In normal conditions, the bone marrow produces cells called myeloblasts which, upon maturation, turn into granulocytes, i.e., cells responsible for defending the body against infections.

In AML, cells from the myeloid line (myeloblasts) proliferate abnormally, progressively invading the bone marrow and interfering with the production of normal blood cells, leading to medullary insufficiency and extramedullary tissue infiltration.

Often, AML is the final stage of other diseases such as chronic myeloproliferative syndromes or myelodysplastic syndromes. The incidence of AML is very high among patients with certain chromosomal abnormalities such as Down syndrome or Fanconi anemia.

AML is a disease of adults, although it can sometimes be observed in children. This type of leukemia represents 40% of all leukemias in the western world. The incidence of AML in Spain is estimated at 15 new cases per million of inhabitants per year.

AML patients have a median age of 64 years old and most patients are between 60 and 75 years old.

AML therefore represents a heterogeneous group of diseases caused by a clonal disorder resulting from genetic abnormalities in hematopoietic stem cells. Most patients have a poor prognosis. In this sense, only between 40% and 55% of adults over 60 years of age achieve complete remission, with long-term survival rates. For younger patients, about 60% to 80% achieve complete remission with the standard treatment. However, only 20% and 30% enjoy a long-term disease-free survival. Despite the new medicinal products that have been designed in recent years, relevant advances in terms of response or survival have not been obtained, and accordingly the standard treatment is still based on the conventional combination of cytarabine and anthracycline. Therefore, the identification of new active compounds in the treatment of this disease is a medical need that is yet to be satisfied.

Multiple myeloma (MM) is the second most common hematological neoplasm. In recent years, therapeutic improvements brought about by immunomodulatory drugs and proteasome inhibitors, among other agents, have allowed a significant progress in the control of this disease. However, MM is still considered incurable. Therefore, considerable efforts are being dedicated to uncover new drugs for improving the therapeutic arsenal available today.

Cannabinoids are the active components of *Cannabis sativa* (marijuana). The therapeutic interests of cannabinoids came following discovery of an endocannabinoid physiological control system in humans, based on cannabinoid receptors (CBs), referred to as CB1 and CB2. While CB1 is extremely abundant in the central nervous system (CNS), CB2 is almost only present in hematopoietic and immune cells, which also express CB1 although to a much lesser extent. Some subsets of hematopoietic cells show high levels of CB2, particularly B-cells, plasma cell precursors. Despite this information, research on the effect of cannabinoids in the hematopoietic system has been less extensive than in the CNS, and many characteristics of the CB2 function and regulation are still rather poorly characterized.

There is increasingly more evidence supporting that cannabinoids may be useful in the treatment of diseases such as glaucoma, osteoporosis, multiple sclerosis, pain, cardiovascular disorders, and neurodegenerative diseases, such as Alzheimer's and Parkinson's diseases. Furthermore, they are used to mitigate vomiting associated with cancer treatment. One of the most exciting therapeutic interests of cannabinoid research lies in its potential antitumor activity. In this sense, some cannabinoids inhibit the proliferation of various tumor cells, such as glioma cell lines, both *in vitro* and *in vivo.* This effect seems to be mediated by the modulation of several signaling pathways involved in cell proliferation, survival, and apoptosis.

MM has an incidence rate of 4-5 per 100,000 inhabitants per year. The age of onset is about 65 years old, and although the therapeutic arsenal has been expanded in recent years with the development of new molecules such as proteosome inhibitors or immunomodulatory drugs (IMIDs), which have added to the conventional treatments such as melphalan and prednisone, as well as hematopoietic progenitor transplant, multiple myeloma is still considered an incurable disease.

In that sense, with the treatments available today, the five-year survival for multiple myeloma is still low, particularly when compared to other types of cancer. Furthermore, there is a need to take into account the side effects of the treatments. For this reason, there is a need to provide alternative treatments with respect to the current ones which do not affect normal cell viability, including the hematopoietic cells from healthy donors post-transplant.

### BRIEF DESCRIPTION OF THE INVENTION

A **first aspect** of the invention relates to the use of a compound, hereinafter compound of the invention, of general formula (I): 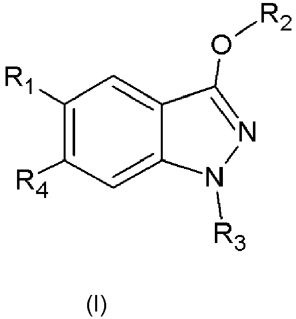
where R1 and R4 are members of the group consisting of hydrogen, halogen, nitro, or amino.
R2 is a member of the group consisting of propyl, butyl, pentyl, cyclohexylmethyl, phenethyl, naphthylmethyl, heterocycloalkyl, primary, secondary or tertiary amine, or substituted benzyl, wherein the phenyl group may contain 1 or 2 substituents of the group consisting of alkyl, hydroxy, methoxy, nitro, amino, or halogen.
R3 is a member of the group consisting of methyl, ethyl, propyl, pentyl, cycloalkylmethyl, cycloalkylethyl, dialkylaminoethyl, heterocycloalkylethyl, cycloalkylcarbonyl (carbonyl group attached to cycloalkyl), heteroarylcarbonyl (carbonyl group attached to heteroaryl), optionally substituted arylcarbonyl (carbonyl group attached to aryl), or optionally substituted aralkylcarbonyl (carbonyl group attached to aralkyl);
or any of the pharmaceutically acceptable salts, esters, tautomers, solvates, and hydrates thereof, or any of the combinations thereof, in the production of a medicinal product for the prevention, relief, improvement, and/or treatment of cancer. More preferably, it relates to the use of a compound of the invention for the production of a medicinal product for the treatment of cancer.

In a preferred embodiment of this aspect, the cancer is a hematological cancer. More preferably, the hematological cancer is acute myeloid leukemia or monoclonal gammopathy. In a more preferred embodiment of this aspect of the invention, the cancer is acute myeloid leukemia. In another more preferred embodiment of this aspect of the invention, the cancer is a monoclonal gammopathy.

In another preferred embodiment of this aspect of the invention:
R1 is a member of the group consisting of hydrogen or amino;
R2 is a member of the group consisting of 4-methoxybenzyl, 1-naphthylmethyl, 2-naphthylmethyl, heterocycloalkyl, diisopropylamino, dimethylamino, diethylamino, piperidinyl, morpholinyl, or pyrrodinyl;
R3 is a member of the group consisting of piperidinoethyl, morpholinoethyl, pyrrolidinylethyl, diisopropylaminoethyl, optionally substituted aryl, optionally substituted aralkyl, 2-thienyl, or 4-chloro-3-pyridyl;
R4 is hydrogen.

In another preferred embodiment:
R1 is a member of the group consisting of hydrogen or amino;
R2 is a member of the group consisting of 4-methoxybenzyl, 1-naphthylmethyl, or 2-naphthylmethyl;
R3 is a member of the group consisting of piperidinoethyl, morpholinoethyl, pyrrolidinylethyl, or diisopropylaminoethyl;
R4 is a member of the group consisting of hydrogen.

In an even more preferred embodiment, the compound of the invention is selected from the list consisting of:
3-(2-naphthylmethoxy)-1-(2-piperidinoethyl)indazole,
3-(1-naphthylmethoxy)-1-(2-piperidinoethyl)indazole,
1-(cyclohexylmethyl)-3-(cyclohexylmethoxy)indazole,
1-methyl-3-(2-naphthylmethoxy)indazole, 1-(2-cyclohexylethyl)-3-(2-naphthylmethoxy)indazole,
1-methyl-3-(3,4-dimethylbenzyloxy)indazole,
3-(2-naphthylmethoxy)-5-nitro-1-(2-piperidinoethyl)indazole,
3-(2-naphthylmethoxy)-5-nitro-1-pentylindazole,
1-methyl-3-(2-naphthylmethoxy)-5-nitroindazole,
1-methyl-5-nitro-3-(phenethoxy)indazole,
5-nitro-1-pentyl-3-(pentyloxy)indazole,
3-(3,4-dimethylbenzyloxy)-1-(2-morpholinoethyl)-5-nitroindazole,
1-methyl-3-(1-naphthylmethoxy)-5-nitroindazole,
1-(2-morpholinoethyl)-3-(2-naphthylmethoxy)-5-nitroindazole,
3-(3,4-dimethylbenzyloxy)-1-methyl-5-nitroindazole,
3-(1-naphthylmethoxy)-1-(2-(1-pyrrolidinyl)ethyl)-5-nitroindazole,
1-(cyclohexylmethyl)-3-(3,4-dimethylbenzyloxy)-5-nitroindazole,
5-bromo-3-(2-naphthylmethoxy)-1-(2-piperidinoethyl)indazole,
1-(2-(diisopropylamino)ethyl)-3-(4-methoxybenzyloxy)indazole,
5-amino-3-(2-naphthylmethoxy)-1-(2-piperidinoethyl)indazole,
3-(4-methoxybenzyloxy)-5-nitro-1-pentylindazole,
3-(2-naphthylmethoxy)-5-nitro-1-propylindazole,
or any of the pharmaceutically acceptable salts, esters, tautomers, solvates, and hydrates thereof, as well as any of the combinations thereof.

Alternatively, the present invention also relates to a compound of general formula (II): 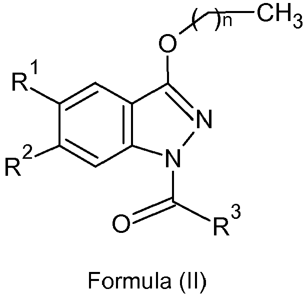 the pharmaceutically acceptable salts, tautomers, prodrugs, solvates, and hydrates thereof, where
- n is selected from 1, 2, 3, and 4;
- R¹ and R² are independently selected from hydrogen, halogen, -NO₂, and - NH₂;
- R³ is selected from cycloalkyl, heteroaryl, optionally substituted aryl, and optionally substituted aralkyl;
- R⁴ is selected from heterocycloalkyl and -NR⁵R⁶;
- R⁵ and R⁶ are independently selected from hydrogen and alkyl.

In a preferred embodiment, the present invention relates to the use of a compound of general formula (II), where R³ is selected from optionally substituted aryl, optionally substituted aralkyl, 2-thienyl, and 4-chloro-3-pyridyl.

In a more preferred embodiment, the present invention relates to the use of a compound of general formula (II), where R³ is selected from 1-naphthyl, 2-naphthyl, 4-tolyl, 3,4,5-trimethylphenyl, 2-benzyloxyphenyl, 3,4,5-trimethoxyphenyl, 2,3-dichlorophenyl, 2,3-difluorophenyl, 2,6-dichlorophenyl, 2,3,6-trifluorophenyl, 2-chlorophenyl, 3-fluorophenyl, 3-chloro-2-fluorophenyl, 4-biphenylol , 4-chlorobenzyl, 4-methoxybenzyl, and 1-adamantyl.

In an even more preferred embodiment, the present invention relates to the use of a compound of general formula (II), where R³ is selected from 1-naphthyl, 2-naphthyl, 2-benzyloxyphenyl, 2,3-dichlorophenyl, and 4-methoxybenzyl.

In a preferred embodiment, the present invention relates to the use of a compound of general formula (II), where R⁴ is selected from heterocycloalkyl, diisopropylamino, dimethylamino, and diethylamino.

In a more preferred embodiment, the present invention relates to the use of a compound of general formula (II), where R⁴ is a heterocycloalkyl.

In an even more preferred embodiment, the present invention relates to the use of a compound of general formula (II), where R⁴ is selected from piperidinyl, morpholinyl, and pyrrolidinyl.

In an even more preferred embodiment, the present invention relates to the use of a compound of general formula (II), where R⁴ is piperidinyl.

In a preferred embodiment, the present invention relates to the use of a compound of general formula (II), where n is selected from 2 and 3.

In a preferred embodiment, the present invention relates to the use of a compound of general formula (II), where R⁴ is a heterocycloalkyl and R³ is selected from 1-naphthyl, 2-naphthyl, and substituted phenyl.

In a more preferred embodiment, the present invention relates to the use of a compound of general formula (II), where R⁴ is a heterocycloalkyl and R³ is selected from 1-naphthyl, 2-naphthyl, 2-benzyloxyphenyl, 2,3-dichlorophenyl, and 4-methoxybenzyl.

In another preferred embodiment, the present invention relates to the use of a compound of general formula (II), where R⁴ is a heterocycloalkyl, R³ is selected from 1-naphthyl, 2-naphthyl, or substituted phenyl, and R¹ and R² are independently selected from hydrogen and halogen.

In a preferred embodiment, the present invention relates to the use of a compound of general formula (I), where R⁴ is -NR₅R₆ and R³ is selected from heteroaryl, optionally substituted aryl, and optionally substituted aralkyl.

In a more preferred embodiment, the present invention relates to the use of a compound of general formula (I), where R⁴ is -NR₅R₆ and R³ is selected from 1-naphthyl, 2-naphthyl, and substituted phenyl.

In a more preferred embodiment, the present invention relates to the use of a compound of general formula (I), where R⁴ is -NR₅R₆, R³ is selected from 1-naphthyl, 2-naphthyl, or substituted phenyl and R¹ and R² are independently selected from hydrogen and halogen.

In a preferred embodiment, the compound of the invention is selected from the list comprising:

| | |
|---|---|
| PGN6 | 3-(1-naphthylmethoxy)-1-(2-piperidinoethyl)indazole |
| PGN17 | 3-(1-naphthylmethoxy)-1-(2-(1-pyrrolidinylethyl)indazole |
| PGN34 | 5-amino-3-(2-naphthylmethoxy)-1-(2-piperidinoethyl)indazole |
| PGN43 | (2,3-dichlorophenyl)(3-(2-(1-pyrrolidinyl)ethoxy)-1-indazolyl)ketone |
| PGN72 | 5-amino-3-(1-naphthylmethoxy)-1-(2-piperidinoethyl)indazole |
| PGN128 | (2,4,6-trimethylphenyl)(3-(3-piperidinopropoxy)-1-indazolyl)ketone |
| PGN152 | (1-naphthyl)(3-(2-piperidinoethoxy)-1-indazolyl)ketone |
| PGN153 | (2-naphthyl)(3-(3-piperidinopropoxy)-1-indazolyl)ketone |

More preferably, the compounds of the invention used in the production of a medicinal product for the prevention, relief, improvement, and/or treatment of a hematological cancer are:
3-(1-naphthylmethoxy)-1-(2-piperidinoethyl)indazole (PGN-6)
3-(1-naphthylmethoxy)-1-(2-(1-pyrrolidinylethyl)indazole (PGN17)
5-amino-3-(2-naphthylmethoxy)-1-(2-piperidinoethyl)indazole (PGN34)
(2,3-dichlorophenyl)(3-(2-(1-pyrrolidinyl)ethoxy)-1-indazolyl)ketone (PGN43)
(2,4,6-trimethylphenyl)(3-(3-piperidinopropoxy)-1-indazolyl)ketone (PGN128), and
(2-naphthyl)(3-(3-piperidinopropoxy)-1-indazolyl)ketone (PGN153)

Even more preferably, the compound is PGN128 (2,4,6-trimethylphenyl)(3-(3-piperidinopropoxy)-1-indazolyl)ketone.

According to the present specification, any of the compounds defined above, i.e., those compounds corresponding to general formula (II), can also be referred to in this specification as "compound or compounds of the invention".

In another preferred embodiment, the compounds of the invention described by formulae (I) and (II) do not include:
1-methyl-3-(1-naphthylmethoxy)-5-nitroindazole (PGN8)
3-(4-methoxybenzyloxy)-5-nitro-1*H*-indazole (PGN37)
3-(1-naphthylmethoxy)-5-nitro-1*H*-indazole (PGN70)

In another preferred embodiment of the first aspect of the invention, the monoclonal gammopathy is selected from multiple myeloma, plasma cell leukemia, Waldenstrom's macroglobulinemia, amyloidosis, or any of the combinations thereof. In an even more preferred embodiment, the monoclonal gammopathy is multiple myeloma.

**A second aspect** of the present invention relates to the use of a composition, hereinafter composition of the invention, comprising or consisting of a compound of the invention, or any of the pharmaceutically acceptable salts, esters, tautomers, solvates, and hydrates thereof, or any of the combinations thereof, for the production of a medicinal product for the prevention, relief, improvement, and/or treatment of cancer.

In a preferred embodiment of this aspect, the cancer is a hematological cancer. More preferably, the hematological cancer is acute myeloid leukemia or monoclonal gammopathy. In a more preferred embodiment of this aspect of the invention, the cancer is acute myeloid leukemia. In another more preferred embodiment of this aspect of the invention, the cancer is a monoclonal gammopathy. In a preferred embodiment of this aspect of the invention, the composition further comprises one or more pharmaceutically acceptable excipients, or consists of a compound of the invention and one or more pharmaceutically acceptable excipients. In another preferred embodiment, the composition further comprises another active ingredient. In a more preferred embodiment, the other active ingredient is selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof. In an even more preferred embodiment, the other active ingredient is dexamethasone. In another even more preferred embodiment, the other active ingredient is melphalan. In another preferred embodiment, the other active ingredient is bortezomib. In another preferred embodiment, the other active ingredient is lenalidomide or thalidomide.

In another preferred embodiment of this second aspect of the invention, the monoclonal gammopathy is selected from multiple myeloma, plasma cell leukemia, Waldenstrom's macroglobulinemia, amyloidosis, or any of the combinations thereof. In a more preferred embodiment, the monoclonal gammopathy is multiple myeloma.

A **third aspect** of the invention relates to a combined preparation, hereinafter combined preparation of the invention, comprising or consisting of:
a) component A which is a compound (compound of the invention) or a composition (composition of the invention) as defined in the present invention, and
b) component B which is an active ingredient selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof.

In an even more preferred embodiment, the active ingredient of (b) is dexamethasone. In another even more preferred embodiment, the active ingredient of (b) is melphalan. In another preferred embodiment, the combined preparation of the invention further comprises pharmaceutically acceptable excipients. In another preferred embodiment, the combined preparation of the invention comprises only those mentioned above as active ingredients, although it may comprise other pharmaceutically acceptable excipients and vehicles.

A fourth aspect relates to the use of the combined preparation of the invention in the production of a medicinal product for simultaneous, separate, or sequential use in therapy. A preferred embodiment of this aspect relates to the use of the combined preparation of the invention, where components A (a) and B (b) are administered simultaneously, separately, or sequentially for the prevention, relief, improvement, and/or treatment of cancer.

In a preferred embodiment of this aspect of the invention, the cancer is a hematological cancer. More preferably, the hematological cancer is acute myeloid leukemia or monoclonal gammopathy. In a more preferred embodiment of this aspect of the invention, the cancer is acute myeloid leukemia. In another more preferred embodiment of this aspect of the invention, the cancer is a monoclonal gammopathy.

In another more preferred embodiment, the monoclonal gammopathy is selected from multiple myeloma, plasma cell leukemia, Waldenstrom's macroglobulinemia, amyloidosis, or any of the combinations thereof. In an even more preferred embodiment, the monoclonal gammopathy is multiple myeloma.

### DESCRIPTION OF THE DRAWINGS

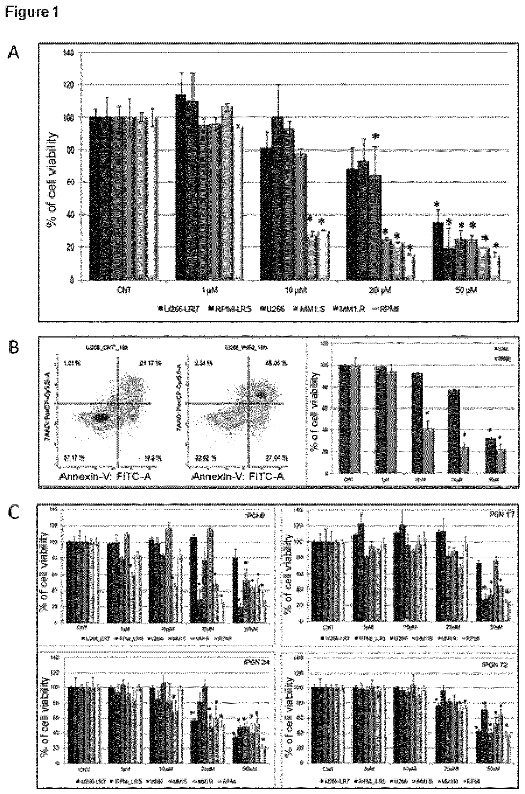

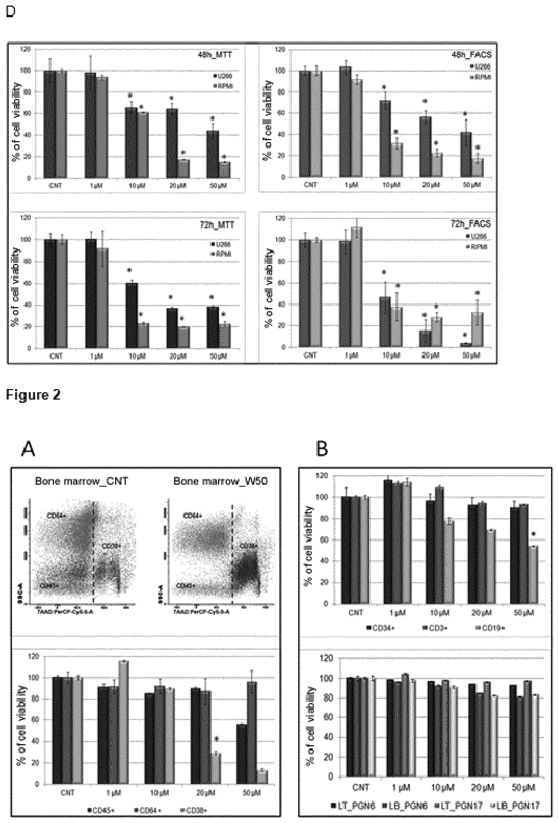

**Figure 1****.** Treatment with different indazole compounds reduces cell viability in different myeloma cell lines. (A) Cell viability analysis by means of MTT assay in U266, RPMI-LR5, U266-LR7, MM1.S, MM1.R, and RPMI following incubation with WIN-55 (0-50 µM) for 18 hours vs. control (CNT). (B) Cell viability determined by flow cytometry using annexin V/7-ADD in U266 and RPMI cell lines following exposure to WIN-55 (0-50 µM) for 18 hours. The DotPlots shown on the left corresponds to a representative analysis for U266 under control conditions (left) and following incubation with 50 µM WIN-55; and the bar graph on the right side of the drawing corresponds to the quantitative analysis resulting from the cytometric analysis of the U266 and RPMI lines. (C) Cell viability analysis by means of MTT assay in the six myeloma cell lines following treatment with different indazole compounds from the PGN family: PGN-6, -17, -34, and -72, tested at the indicated doses for 18 hours. The data represents the mean ± SD of three experiments in triplicate. Statistical significance*: *p=0.05* with respect to the control conditions (untreated, CNT). See Table 2 for IC50 values. (D) Cell viability following long-term, i.e., 48- and 72-hour, treatment with WIN-55 in the more resistant and sensitive, U266 and RPMI, myeloma cell lines. Cell viability determined by means of MTT assay after 48 hours (graph on top) and after 72 hours (graph at the bottom) is shown on the left. Cell viability analyzed by means of flow cytometry after 48 hours (graph on top) and after 72 hours (graph at the bottom) is shown on the right side of the panel. The data represents the mean ± SD of three independent experiments in triplicate.
**Figure 2****.** Selective effect of WIN-55 on the myelomatous cells of patients, while the normal cells of healthy individuals are not affected. (A) Bone marrow cells (BM cells) isolated from 6 MM patients were treated with WIN-55 (0-50 µM) for 18 hours. The different populations of BM were stained with 7AAD and a suitable combination of antibodies for identifying granulomonocytic cells (CD64+), lymphocytic cells (CD45+), and myelomatous cells (CD38+). The top panel shows the cytometry DotPlot representative of a patient corresponding to the control conditions (CNT, left) and BM cells treated with 50 µM of WIN-55 (W50, right). The bottom panel shows the graph corresponding to the data obtained from the quantitative analysis of the BM cells of all MM patients (n=6). (B) Peripheral blood cells (PB cells) of healthy donors were immunogenetically classified using CD34+, CD3+, and CD19+ microbeads for isolating hematopoietic stem cells, T-cells, and B-cells, respectively. The top panel shows the cell viability of the hematopoietic stem cells (CD34+), lymphocytic T-cells (CD3+), and B-cells (CD19+) following treatment with WIN-55 (0-50 µM) for 18 hours analyzed by means of MTT assay. The bottom panel shows the cell viability in lymphocytic T-cells (LT, CD3+) and B-cells (LB, CD19+) following treatment with two indazole compounds of the PNG family, PGN-6 and -17. The data represents the mean ± SD of three experiments in triplicate. Statistical significance*: *p=0.05* with respect to the control conditions (CNT, untreated).

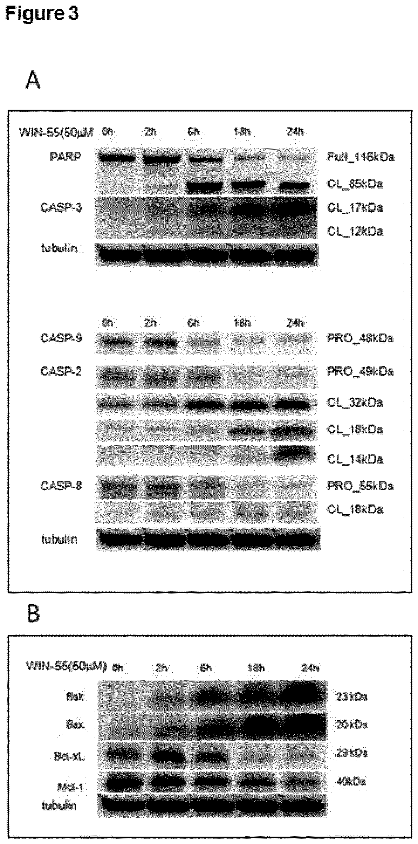

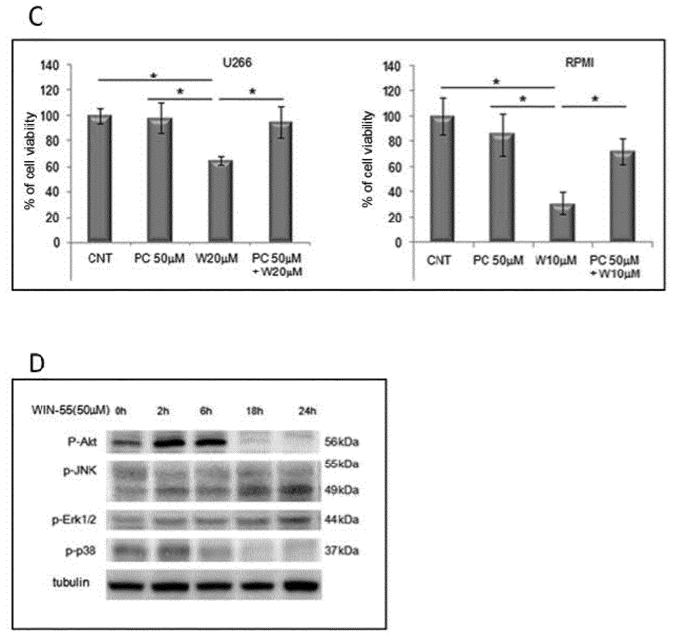

**Figure 3****.** The antiproliferative effect of WIN-55 is mediated primarily by caspase-dependent apoptosis mechanisms and by the Akt transduction signaling pathway. U266, the most resistant cell line, was treated with 50 µM of WIN-55 in the indicated times. (A) Western-blot of PARP forms (full FULL and cleaved CL) and whole (PRO, pro-form) and cleaved (CL, cleaved) Casp-3, -9, -2, and -8. (B) Western-blot of proteins of the Bcl-2, Bak, Bax, Bcl-xL, and Mcl-1 family. (C) Cell viability of U266 and RPMI cell lines following incubation with the pan-caspase inhibitor ZVAD-FMK (PC) and/or WIN-55 (W) for 18 hours at the indicated concentrations, analyzed by means of MTT assay. The data represents the mean ± SD of three independent experiments in triplicate. Statistical significance*: *p*=*0.05* with respect to cells treated with 20 µM of WIN-55 for U266 and 10 µM for the RPMI cell line. (D) Effect of the compound of the invention on the Akt, Erk, JNK, and p38 signaling pathways evaluated by means of Western blot on extracts of U266 cells incubated with 50 µM of WIN-55 in the indicated times. Tubulin was used as load control.

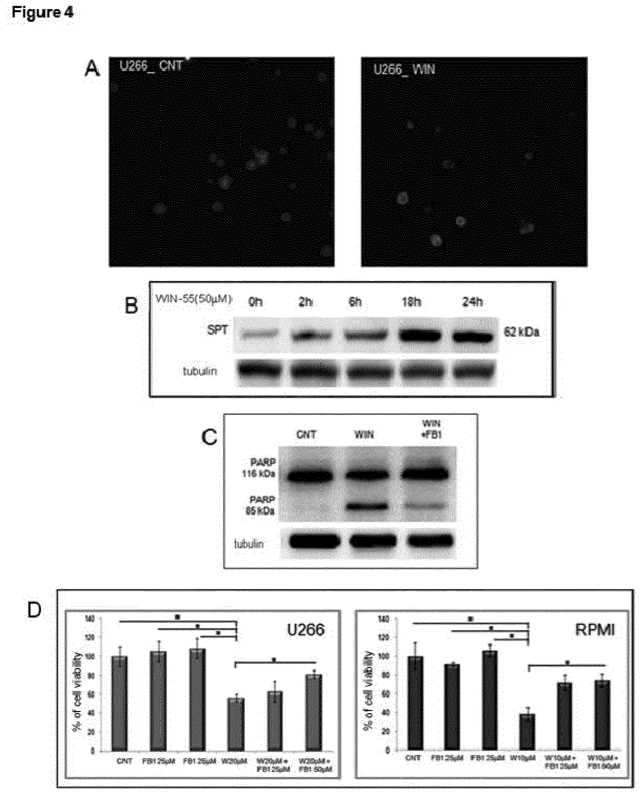

**Figure 4****.** WIN-55 induces ceramide synthesis in MM cells. (A) Immunohistochemical detection of ceramide in untreated U266 cells (CNT, left) and following treatment with 50 µM of WIN-55 for 6 hours (WIN, right). (B) Western-blot of SPT, the enzyme limiting the rate of ceramide synthesis, in U266 cells treated with 50 µm of WIN-55 for the indicated times. (C) PARP expression levels evaluated by means of Western blot in cells treated with WIN-55 (WIN) and with/without 50 µM of fumonisin B1 (FB1). (D) Cell viability analysis using MTT assay in U266 cells (left) and RPMI cells (right) treated with WIN-55 (W), fumonisin B1 (FB1), or a combination of both for 18 hours, at the indicated doses. The data represents the mean ± SD of three independent experiments in triplicate. Statistical significance*: *p=0.05* with respect to cells treated with 20 µM of WIN-55 for U266 and 10 µM for the RPMI cell line. Tubulin was used as load control.

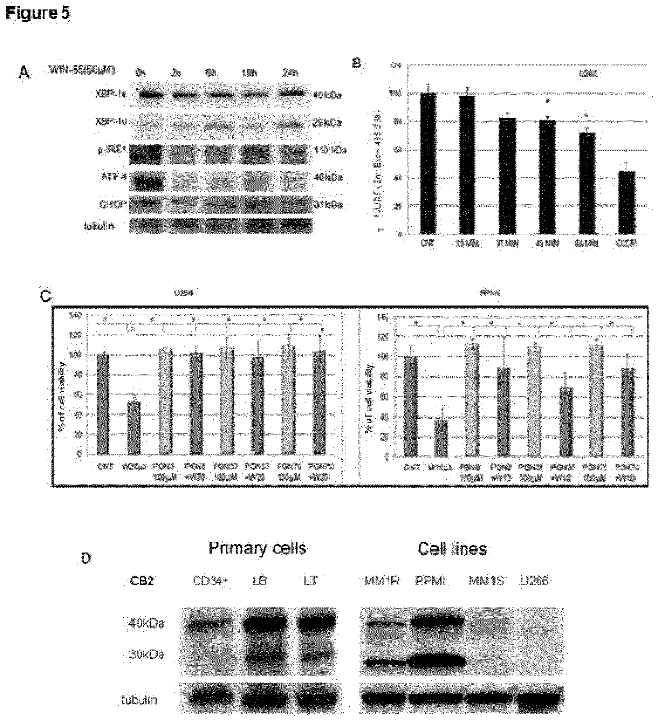

**Figure 5****.** WIN-55 attenuates the stress response of the basal endoplasmic reticulum in U266 cells and promotes an early loss of mitochondrial membrane potential. (A) Western-blot of the proteins involved in the unfolded protein response (UPR), such as CHOP, ATF-4, p-IRE1, and XBP-1s and XBP-1u following treatment with 50 µM of WIN-55 in the specified points in time. (B) Loss of mitochondrial membrane potential in U266 cells following treatment with 50 µM of WIN-55 in the indicated times, incubation medium with DMSO<0.15% was used as control (CNT), and CCCP was used as positive control of the loss of potential. The data represents the mean ± SD of three independent experiments in triplicate. (C) Cell viability determined by means of MTT assay following treatment with WIN-55 and/or CB2-specific cannabinoid antagonists: PGN-8, PGN-37, and PGN-70 at 100 µm for U266 and 50 µm for RPMI. The data represents the mean ± SD of three independent experiments in triplicate. Statistical significance*: *p=0.05* with respect to cells treated with 20 µM of WIN-55 for U266 (W20) and 10 µM for the RPMI cell line (W10). (D) Expression pattern of the CB2 receptor in several cell lines and primary cells of healthy individuals (hematopoietic stem cells, lymphocytic T-cells, and B-cells) determined by Western blot. The 40 kDa band corresponds to the complete monomeric form of the CB2 receptor and the 30 kDa band corresponds to the truncated form. Tubulin was used as load control.

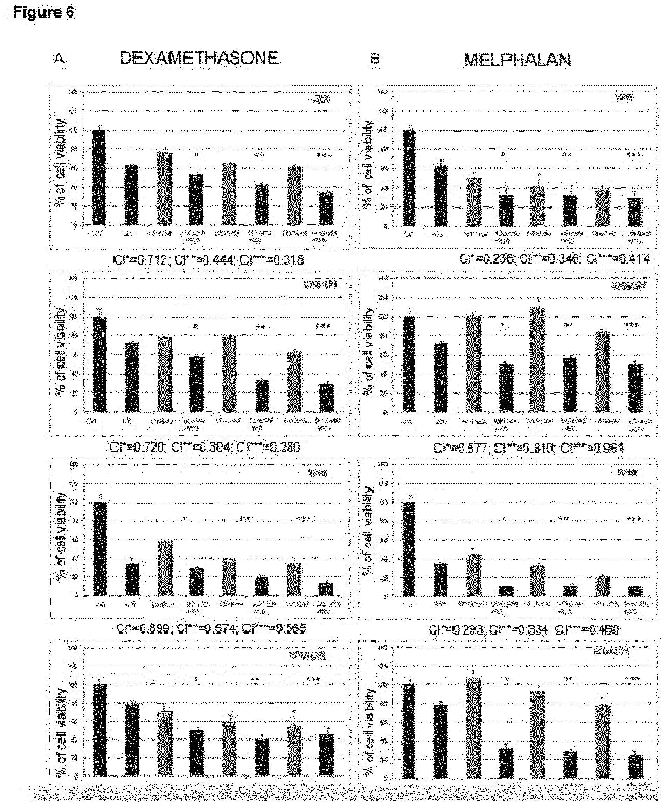

**Figure 6****.** WIN-55 synergizes with other anti-myeloma agents. Determination of cell viability in U266, U266-LR7, RPMI, and RPMI-LR5 using MTT assay following treatment with a fixed dose of WIN-55 (W; 20 µm for U266, U266-LR7, and RPMI-LR5, and 10 µm for RPMI), which was below the IC50 corresponding to each cell line tested according to Table 2, in combination with increasing concentrations of dexamethasone (DEX, between 5 µM and 20 µm) (panel A) or melphalan (MPH, between 1 µM and 4 µM for U266 and U266-LR7 lines; and between 0.05 µM and 0.5 µM for RPMI and RPMI-LR5 lines) (panel B). The asterisks indicate the values of the combination index (CI) which correspond to the combination and are shown below each graph.

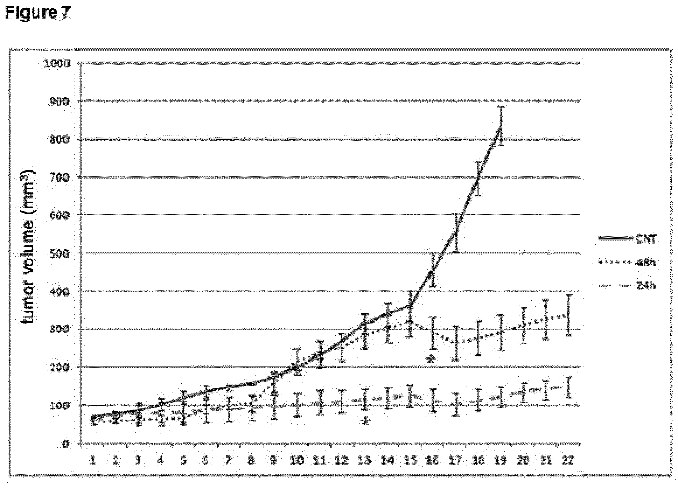

**Figure 7****.** WIN-55 considerably inhibits tumor growth in NGS mice *in vivo.* 5 x 10⁶ U266 cells were inoculated subcutaneously in the interscapular flank and the mice were randomly distributed into three groups (n=10) for receiving 5 mg/kg i.p. of WIN-55 every 24 hours, every 48 hours, and the vehicle (<0.15% of DMSO in the medium) as positive control group. The diameter of the tumors was measured every two days and the volume was estimated as the volume of the ellipse. The graph shows the progression of tumor volume for the indicated days. Statistical significance was defined as p≤0.05 and the asterisk indicates the first day in which the differences were statistically significant for each dose, i.e., day 13 for the group treated every 24 hours and day 16 for the group treated every 48 hours. The mice from the control group (CNT) were sacrificed on day 19 for ethical reasons. The data represents the mean ± SD of volume of all the mice in each group.

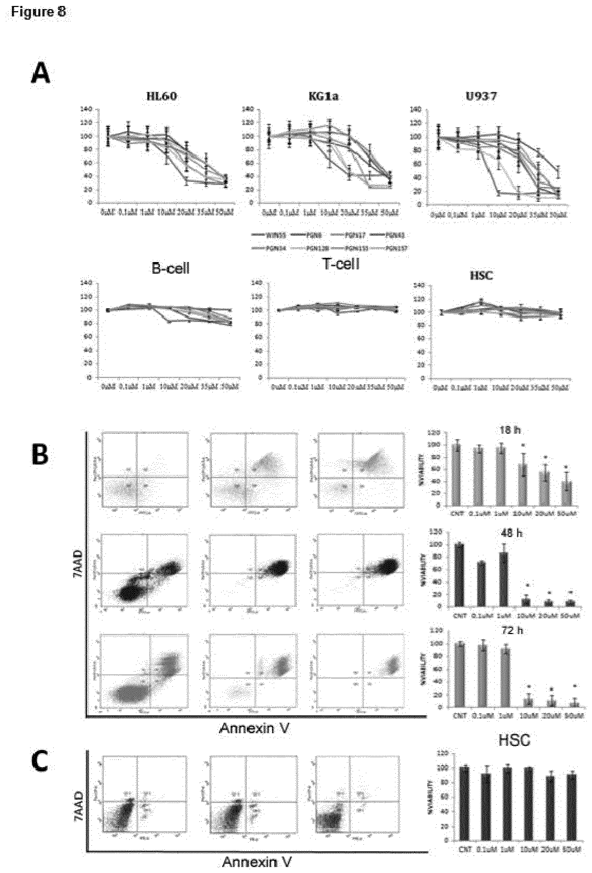

**Figure 8****.** The viability of AML cell lines decreases significantly following cannabinoid treatment, while the viability of healthy cells, such as CD34+ hematopoietic stem cells, is not affected. (A) Three AML cell lines (HL60, KG-1a, and U937) and three healthy cell (HSC cell, B-cell, and T-cell) populations were treated with increasing concentrations of cannabinoid WIN-55.212-2 and the PGN family for 18 hours, and cell viability was analyzed by means of WST-1 assay. (**B**) The DotPlot corresponds to the viability analysis of the HL60 cells using flow cytometry after 18, 48, and 72 hours. Only the control, 20 µM and 50 µM of WIN-55.212-2, are shown, the results of all the cannabinoids for all doses and times are, however, not shown. (**C**) HSC (CD34+) populations were also analyzed by flow cytometry. In all the cases, the mean values of the proliferation of untreated control samples were taken as 100%. The results are representative of four experiments in triplicate. * indicates significant differences in the value of p ≤0.05.

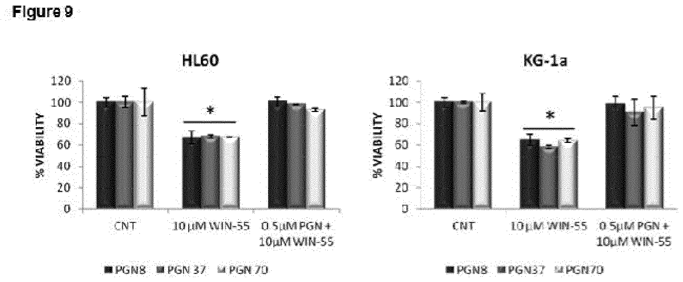

**Figure 9****.** HL60 and KG-1a cells were incubated with WU-55.212-2 10 µM in the presence of selective vehicles or antagonists of CB2. After 18 hours, the number of viable cells was determined by WST-1 assay. In all the cases, the mean values of the proliferation of untreated control samples were taken as 100%. The results are representative in four experiments performed in triplicate. * indicates significant differences in the value of p ≤0.05.

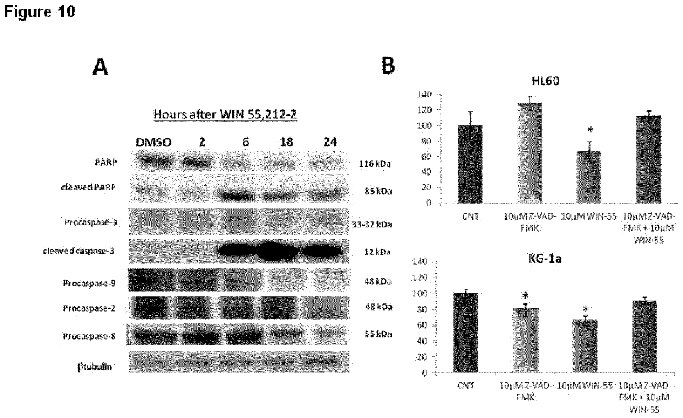

**Figure 10****.** The antiproliferative effect of cannabinoids on AML cells is mediated by apoptotic mechanisms. (**A**) HL60 cells were incubated with 50 µM of WIN-55,212-2 at the indicated times and the expression of the cleaved forms of the executioner caspase, Casp-3, and the fragmentation of its substrate PARP were analyzed by means of Western blot. The expression of the main initiator caspases, Casp-9, Casp-2, and Casp-8, is also shown. Three experiments were performed, and for each experiment, four gels and electroblots were performed simultaneously, improving reproducibility. One in every four gels was used for detecting tubulin as load control.
   (**B**) The proapoptotic effect of cannabinoids in AML cells was abolished following co-culture with pan-caspase inhibitors. HL60 and KG-1a cells were incubated with 10 µM of WIN-55,212-2 in the presence of pan-caspase inhibitor Z-VAD(OMe)-FMK or a vehicle. After 18 hours, the number of viable cells was determined by WST-1 assay. In all the cases, the mean values of the proliferation of untreated control samples were taken as 100%. The results are representative of four experiments performed in triplicate. * indicates significant differences in the value of p ≤0.05.

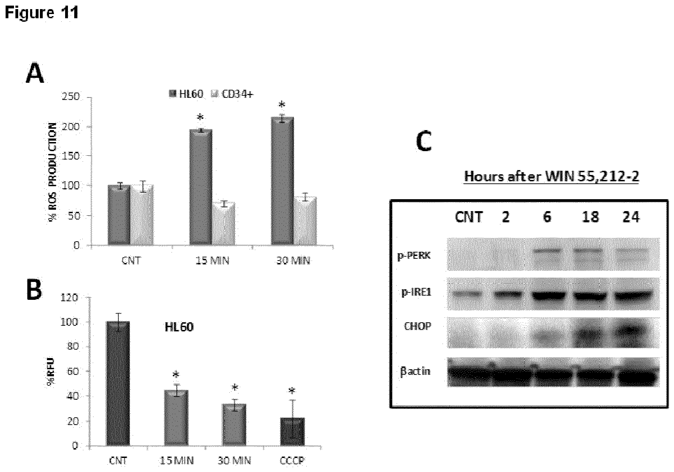

**Figure 11****.** WIN-55,212-2 promotes early mitochondrial damage and ER stress. HL60 cells untreated and treated (10⁶ cells per assay) with WIN-55,212-2 (50 µM) for 15 and 30 minutes at 37°C were stained with (**A**) TMRE to evaluate mitochondrial membrane potential using a "Fluoroscan" multiwell plate reader. For each condition, triplicate samples were prepared (at least five times). CCCP (2-[2-(3-chlorophenyl)hydrazinylidene]propanedinitrile) was used as positive control for the loss of Δψm. (**B**) A 5 µM MitoSOX probe was used for detecting mitochondrial superoxide. The MitoSOX signal was detected using flow cytometry. Mitochondrial superoxide in HSC was also detected (C). The expression of three proteins involved in the unfolded protein response was analyzed by means of Western blot in HL60 cells treated with 50 µM of HL60 in the indicated moments. * indicates significant differences in the value of p ≤0.05.

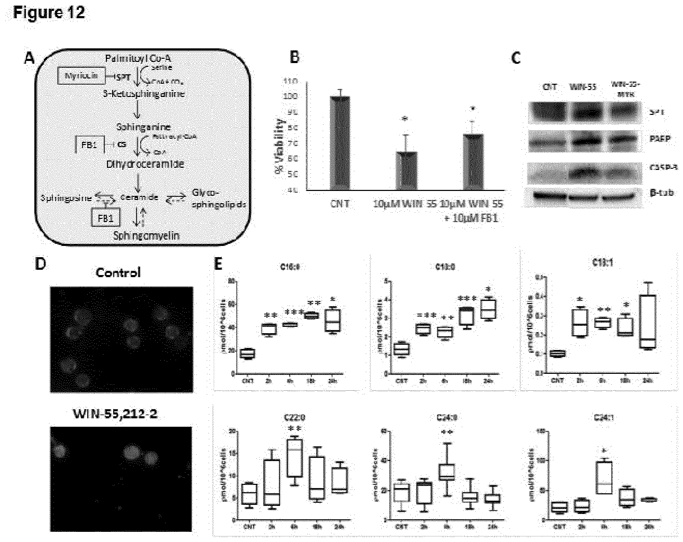

**Figure 12****.** The accumulation of ceramides is involved in cannabinoid-induced apoptosis. (**A**) Diagram of the main inhibitors of *de novo* ceramide synthesis. (**B**) HL60 cells were incubated with 10 µM of WIN-55,212-2 in the presence of fumonisin B1 or a vehicle. After 18 hours, the number of viable cells was determined by WST-1 assay. In all the cases, the mean values of the proliferation of untreated control samples were taken as 100%. The results are representative of four experiments performed in triplicate. * indicates significant differences in the value of p ≤0.05. (**C**) HL60 cells were incubated with 10 µM of WIN-55,212-2 in the presence of myriocin or a vehicle. The expression of SPT, PARP, and Casp-3 was analyzed by Western blot. (**D**) Staining photomicrograph of ceramides in HL60 cells not subjected to control (control) and treated with 50 µM of WIN-55,212-2 for 18 hours. (**E**) HL60 cells were incubated with 50 µM of WIN-55,212-2 in the indicated moments and the levels of different types of ceramides were quantified by means of HPLC/MS-MS using calibration curves in which ceramide 17:0 was used as internal standard. The ceramide content was directly proportional to the ceramide/internal standard ratio (r>0.99, p<0.01). The relative standard deviations (RSD) were <10%.

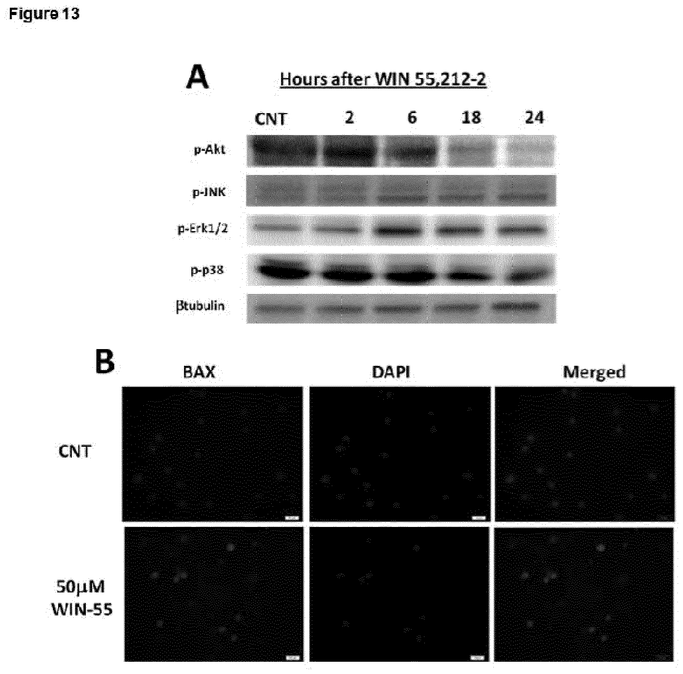

**Figure 13****.** Signaling pathways signaled by cannabinoids in AML cells. In the top part of the panel, HL60 cells were treated in the indicated moments with 50 µM of WIN-55,212-2. (**A**) Different signaling pathways, such as MAPK and Akt, were analyzed using Western blot. (**B**) The expression of Bax was measured by means of immunocytofluorescence analysis.

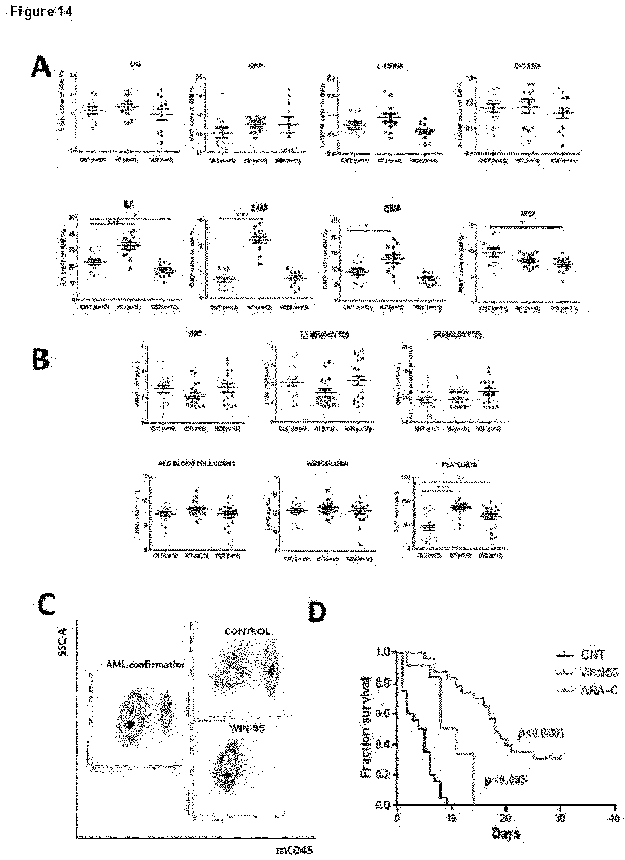

**Figure 14****.** The anti-tumor effect of cannabinoids in murine AML models *in vivo.* Healthy BALB/c mice were treated with a vehicle or WIN-55,212-2 at a dose of 5 mg/kg/day for 7 and 28 days. (**A**) Populations of bone marrow cells were analyzed by flow cytometry, and (**B**) populations of peripheral blood cells were analyzed by blood count studies. NOD/scid/IL-2R gammae null (NSG) mice were monitored to confirm disease progression by studying the detection of human CD45+ cells in the bone marrow (BM) by means of BM aspirates and flow cytometry assays. Once the presence of leukemia cells was confirmed, treatment with a vehicle or cannabinoid WIN-55,212-2 was administered at a dose of 5 mg/kg/day. (**D**) The survival of mice treated with 5 mg/kg/day of cannabinoid WIN-55,212-2 was compared with the control group and the group treated with 50 mg/kg of ARA-C for 5 days. More than 20 mice per group were used.

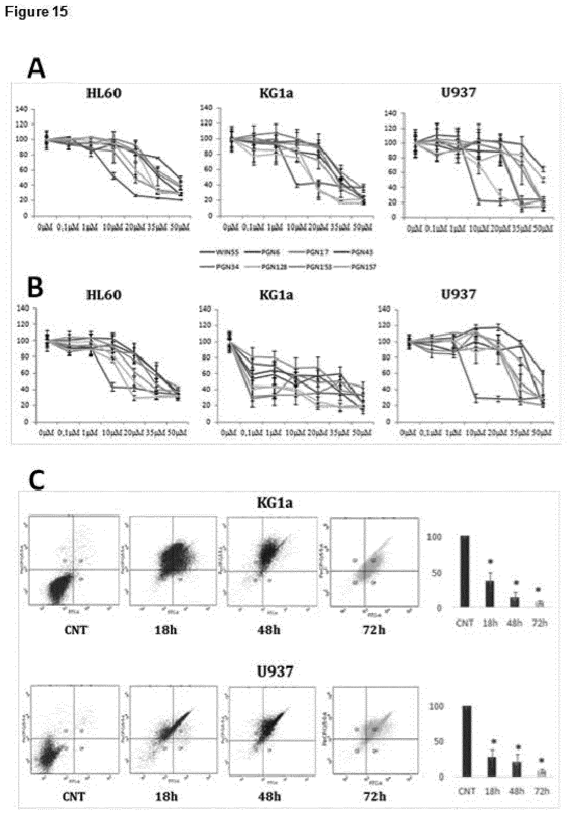

**Figure 15****.** The viability of AML cell lines decreased significantly following treatment with cannabinoids in different moments. (**A**) HL60, KG-1a, and U937 cell lines were treated for 48 hours (**B**) and 72 hours with increasing concentrations of cannabinoids WIN-55.212-2 and of PGN family (compounds of the invention, formula I and formula II). Cell viability was analyzed by means of WST-1 assay. (C) The DotPlot corresponds to the viability analysis of KG-1a and U937 cells using flow cytometry after 18, 48, and 72 hours. Only untreated cells and cells treated with 50 µM of WIN-55.212-2 are shown, the results of all the cannabinoids for all doses and times are, however, not shown. In all the cases, the mean values of the proliferation of the untreated control samples were taken as 100%. The results are representative of four experiments in triplicate. * indicates significant differences in the value of p ≤0.05.

### DETAILED DESCRIPTION OF THE INVENTION

The authors of the present invention describe for the first time the use of indazole compounds of the present invention for the prevention, relief, improvement, and/or treatment of cancer, preferably hematological cancer, more preferably acute myeloid leukemia or monoclonal gammopathy, and even more preferably, among monoclonal gammopathies, those selected from multiple myeloma, plasma cell leukemia, Waldenstrom's macroglobulinemia, amyloidosis, or any of the combinations thereof. Particularly, the monoclonal gammopathy is multiple myeloma. Multiple myeloma (MM) is a neoplasm which is characterized by clonal proliferation of malignant plasma cells in the bone marrow and is associated with the presence of monoclonal component or protein M in blood and/or serum.

The therapeutic improvements brought about by immunomodulatory drugs such as lenalidomide and proteosome inhibitors such as bortezomib, among other agents, have allowed a significant progress in the control of this disease. However, MM is still considered incurable. In order to achieve an effective therapy against this disease, the authors of the present invention have developed and tested indazole compounds of synthetic origin which have an anti-tumor effect.

Specifically, it is demonstrated that the different compounds under studied induce selective apoptosis in myeloma cell lines and plasma cells in the first stage of malignancy in MM patients, without affecting the viability of the normal cells of healthy donors, including hematopoietic stem cells. This antiproliferative effect is mediated by the activation of caspases, mainly caspase 2, and partially prevented by a pan-caspase inhibitor. Indazole compound-induced apoptosis correlated with an increase in the expression of Bax and Bak and a decrease in Bcl-xL and Mcl-1. Furthermore, treatment with indazole compounds induced a biphasic Akt/PKB response and significantly increased the levels of ceramide in MM cells. Surprisingly, blocking the synthesis of ceramide prevented indazole compound-induced apoptosis, which indicates that ceramides play a key role in the pro-apoptotic effect of said compounds in MM cells. Furthermore, blocking the cannabinoid receptor CB2 also inhibited indazole compound-induced apoptosis. The compound WIN-55 increased the anti-myeloma activity of dexamethasone and melphalan, overcoming cell resistance to melphalan *in vitro.* Finally, the administration of the cannabinoid WIN-55 to a plasmacytoma mouse model significantly suppressed tumor growth *in vivo.*

The authors of the present invention have observed a dramatic anti-leukemia effect with specific CB2 agonists which was completely reverted when the cells were co-incubated with antagonists of CB2 receptors, an intriguing data compared to other authors who suggest that CB2 may be a proto-oncogene that is involved in leukemogenesis because, when it is overexpressed in myeloid precursors, Cb2 induces blockade of neutrophilic development and stimulates the migration of Cb2-expressing cells *in vitro.*

In the present invention, the inventors show a new family of CB2 cannabinoid-specific derivatives which reduce the viability of AML cells. Furthermore, this effect was highly selective, given that the viability of normal healthy cells, including hematopoietic stem cells, remained unaffected. Furthermore, the data demonstrates that a synthetic agonist of the cannabinoid receptor, WIN 55,212-2, potently and in a dose-dependent manner induced by the apoptotic cell death of AML cells.

All in all, the data suggests that these indazole compounds can be considered therapeutic agents in the treatment of cancer, preferably a hematological cancer, more preferably in the treatment of acute myeloid leukemia or a monoclonal gammopathy, and specifically, among monoclonal gammopathies, multiple myeloma.

The present invention therefore relates to the use of indazole derivatives in the production of a medicinal product for the prevention, relief, improvement, and/or treatment of a monoclonal gammopathy, preferably multiple myeloma.

### MEDICAL USE OF THE COMPOUND OF THE INVENTION

Therefore, a **first aspect** of the invention relates to the use of a compound, hereinafter compound of the invention, of general formula (I): 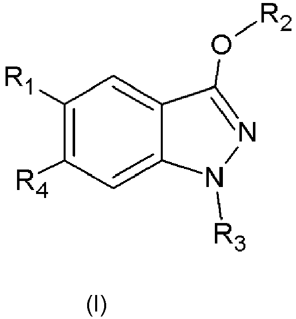 where
- R1 and R4 are members of the group consisting of hydrogen, halogen, nitro, or amino.
- R2 is a member of the group consisting of propyl, butyl, pentyl, cyclohexylmethyl, phenethyl, naphthylmethyl, heterocycloalkyl, primary, secondary or tertiary amine, or substituted benzyl, wherein the phenyl group may contain 1 or 2 substituents of the group consisting of alkyl, hydroxy, methoxy, nitro, amino, or halogen.
- R3 is a member of the group consisting of methyl, ethyl, propyl, pentyl, cycloalkylmethyl, cycloalkylethyl, dialkylaminoethyl, heterocycloalkylethyl, cycloalkylcarbonyl (carbonyl group attached to cycloalkyl), heteroarylcarbonyl (carbonyl group attached to heteroaryl), optionally substituted arylcarbonyl (carbonyl group attached to aryl), or optionally substituted aralkylcarbonyl (carbonyl group attached to aralkyl);
or any of the salts thereof, preferably any pharmaceutically acceptable salt, pharmaceutically acceptable esters, tautomers, polymorphs, hydrates, or an isomer, prodrugs, derivatives, solvates, or analogs thereof, or any of the combinations thereof, hereinafter compound of the invention, in the production of a medicinal product for the prevention, relief, improvement, and/or treatment of cancer. Alternatively, it relates to the compound of the invention for use in the prevention, relief, improvement, and/or treatment of cancer.

In a preferred embodiment of this aspect, the cancer is a hematological cancer. More preferably, the hematological cancer is acute myeloid leukemia or monoclonal gammopathy. In a more preferred embodiment of this aspect of the invention, the cancer is acute myeloid leukemia. In another more preferred embodiment of this aspect of the invention, the cancer is a monoclonal gammopathy.

In a preferred embodiment of this aspect of the invention:
- R1 is a member of the group consisting of hydrogen or amino;
- R2 is a member of the group consisting of 4-methoxybenzyl, 1-naphthylmethyl, 2-naphthylmethyl, heterocycloalkyl, diisopropylamino, dimethylamino, diethylamino, piperidinyl, morpholinyl, or pyrrodinyl;
- R3 is a member of the group consisting of piperidinoethyl, morpholinoethyl, pyrrolidinylethyl, diisopropylaminoethyl, optionally substituted aryl, optionally substituted aralkyl, 2-thienyl, or 4-chloro-3-pyridyl;
- R4 is hydrogen.

In another preferred embodiment:
- R1 is a member of the group consisting of hydrogen or amino;
- R2 is a member of the group consisting of 4-methoxybenzyl, 1-naphthylmethyl, or 2-naphthylmethyl;
- R3 is a member of the group consisting of piperidinoethyl, morpholinoethyl, pyrrolidinylethyl, or diisopropylaminoethyl;
- R4 is hydrogen.

In an even more preferred embodiment, the compound of the invention is selected from the list consisting of:
3-(2-naphthylmethoxy)-1-(2-piperidinoethyl)indazole,
3-(1-naphthylmethoxy)-1-(2-piperidinoethyl)indazole,
1-(cyclohexylmethyl)-3-(cyclohexylmethoxy)indazole,
1-methyl-3-(2-naphthylmethoxy)indazole,
1-(2-cyclohexylethyl)-3-(2-naphthylmethoxy)indazole,
1-methyl-3-(3,4-dimethylbenzyloxy)indazole,
3-(2-naphthylmethoxy)-5-nitro-1-(2-piperidinoethyl)indazole,
3-(2-naphthylmethoxy)-5-nitro-1-pentylindazole,
1-methyl-3-(2-naphthylmethoxy)-5-nitroindazole,
1-methyl-5-nitro-3-(phenethoxy)indazole,
5-nitro-1-pentyl-3-(pentyloxy)indazole,
3-(3,4-dimethylbenzyloxy)-1-(2-morpholinoethyl)-5-nitroindazole,
1-methyl-3-(1-naphthylmethoxy)-5-nitroindazole,
1-(2-morpholinoethyl)-3-(2-naphthylmethoxy)-5-nitroindazole,
3-(3,4-dimethylbenzyloxy)-1-methyl-5-nitroindazole,
3-(1-naphthylmethoxy)-1-(2-(1-pyrrolidinyl)ethyl)-5-nitroindazole,
1-(cyclohexylmethyl)-3-(3,4-dimethylbenzyloxy)-5-nitroindazole,
5-bromo-3-(2-naphthylmethoxy)-1-(2-piperidinoethyl)indazole,
1-(2-(diisopropylamino)ethyl)-3-(4-methoxybenzyloxy)indazole,
5-amino-3-(2-naphthylmethoxy)-1-(2-piperidinoethyl)indazole,
3-(4-methoxybenzyloxy)-5-nitro-1-pentylindazole,
3-(2-naphthylmethoxy)-5-nitro-1-propylindazole,
or any of the salts thereof, preferably any pharmaceutically acceptable salt, pharmaceutically acceptable esters, tautomers, polymorphs, hydrates, or an isomer, prodrugs, derivatives, solvates, or analogs thereof, or any of the combinations thereof.

In another preferred embodiment of the first aspect of the invention, the monoclonal gammopathy is selected from the list consisting of multiple myeloma, plasma cell leukemia, Waldenstrom's macroglobulinemia, amyloidosis, or any of the combinations thereof. In a more preferred embodiment, the monoclonal gammopathy is multiple myeloma.

In this specification, when mention is made to the term "pharmaceutically and/or physiologically acceptable salts or solvates," it refers to any pharmaceutically acceptable salt, ester, solvate, or any other compound which, when administered, is capable of providing (directly or indirectly) a compound such as those described herein. Nevertheless, it will observed that non-pharmaceutically acceptable salts also fall within the scope of the invention, because they may be useful for the preparation of pharmaceutically acceptable salts. The preparation of salts, prodrugs, and derivatives may be carried out by means of methods known in the state of the art.

For example, the pharmaceutically acceptable salts of the compounds provided herein are synthesized from the compound of the invention by means of conventional chemical methods. Such salts are generally prepared, for example, by reacting free acid or base forms of these compounds with a stoichiometric amount of the suitable base or acid in water, or in an organic solvent, or in a mixture of both. Generally, non-aqueous media such as ether, ethyl acetate, ethanol, isopropanol, or acetonitrile are preferred. Examples of acid addition salts include mineral acid addition salts such as, for example, hydrochloride, hydrobromide, hydroiodide, sulfate, nitrate, phosphate, and organic acid addition salts such as, for example, acetate, maleate, fumarate, citrate, oxalate, succinate, tartrate, malate, mandelate, methanesulfonate, and p-toluenesulfonate.

Examples of base addition salts include inorganic salts such as, for example, sodium, potassium, calcium, ammonium, magnesium, aluminum, and lithium, and organic base salts such as, for example, ethylenediamine, ethanolamine, N,N-dialkylenethanolamine, glucamine, and basic amino acid salts.

The compounds of the present invention represented by formula (I) may include isomers, depending on the presence of multiple bonds, including optical isomers or enantiomers, depending on the presence of chiral centers. The individual isomers, enantiomers, or diastereoisomers and the mixtures thereof fall within the scope of the present invention, i.e., the term isomer also refers to any mixture of isomers, such as diastereomers, racemic isomers, etc., even the optically active isomers thereof or the mixtures thereof in different proportions. The individual enantiomers or diastereoisomers, as well as the mixtures thereof, can be separated by means of conventional techniques.

Likewise, the prodrugs of the compounds of formula (I) fall within the scope of this invention. As it is used herein, the term "prodrug" includes any derivative of a compound of formula (I), for example and in a non-limiting manner: esters (including carboxylic acid esters, amino acid esters, phosphate esters, sulfonate esters of metal salts, etc.), carbamates, amides, etc., which when administered to an individual can be transformed directly or indirectly into said compound of formula (I) in the mentioned individual. Advantageously, said derivative is a compound which increases the bioavailability of the compound of formula (I) when administered to an individual, or it enhances the release of the compound of formula (I) in a biological compartment. The nature of said derivative is not critical provided that it can be administered to an individual and can provide the compound of formula (I) in a biological compartment of an individual. The preparation of said prodrug can be carried out by means of conventional methods known by those skilled in the art.

As it is used herein, the term "derivative" includes both pharmaceutically acceptable compounds, i.e., derivatives of the compound of formula (I) which can be used in the production of a medicinal product or food compositions, and non-pharmaceutically acceptable derivatives because they may be useful in the preparation of pharmaceutically acceptable derivatives.

The compounds of the invention can be in a crystalline form as free compounds or solvates. In this sense, as it is used herein the term "solvate" includes both pharmaceutically acceptable solvates, i.e., solvates of the compound of formula (I) which can be used in the production of a medicinal product, and non-pharmaceutically acceptable solvates, which may be useful in the preparation of pharmaceutically acceptable salts or solvates. The nature of the pharmaceutically acceptable solvate is not critical provided that it is pharmaceutically acceptable. In a particular embodiment, the solvate is a hydrate. The solvates can be obtained by conventional solvation methods known by those skilled in the art.

For their application in therapy, the compounds of formula (I), the salts, prodrugs, or solvates thereof, will preferably be in a pharmaceutically acceptable or substantially pure form, i.e., having a pharmaceutically acceptable level of purity, excluding normal pharmaceutical additives such as diluents and carriers, and not including material considered toxic at normal dosage levels. The levels of purity for the active ingredient are preferably greater than 50%, more preferably greater than 70%, and even more preferably greater than 90%. In a preferred embodiment, they are greater than 95% of the compound of formula (I), or of the salts, solvates, or prodrugs thereof.

Particularly preferred derivatives or prodrugs are those which increase the bioavailability of the compounds of the invention when they are administered to the subject (for example, allowing an orally administered compound to be absorbed more quickly, accelerating its passage to the blood) or improve the supply of the compound to a biological compartment (for example, the brain or lymphatic system) with respect to the initial compound.

### PHARMACEUTICAL COMPOSITION AND DOSAGE FORM OF THE INVENTION

A **second aspect** of the present invention relates to the use of a composition, hereinafter composition of the invention, comprising or consisting of a compound of the invention, in the production of a medicinal product for the prevention, relief, improvement, and/or treatment of cancer. Alternatively, it relates to the composition of the invention for use in the prevention, relief, improvement, and/or treatment of cancer.

In a preferred embodiment of this aspect, the cancer is a hematological cancer. More preferably, the hematological cancer is acute myeloid leukemia or monoclonal gammopathy. In a more preferred embodiment of this aspect of the invention, the cancer is acute myeloid leukemia. In another more preferred embodiment of this aspect of the invention, the cancer is a monoclonal gammopathy.

In a preferred embodiment of this aspect of the invention, the composition further comprises one or more pharmaceutically acceptable excipients or vehicles. Preferably, the composition of the invention is a pharmaceutical composition comprising as the only active ingredient a compound of the invention, although it may comprise one or more pharmaceutically acceptable excipients and/or vehicles. In another preferred embodiment, the composition further comprises another active ingredient. In a more preferred embodiment, the other active ingredient is selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof. In an even more preferred embodiment, the other active ingredient is dexamethasone. In another even more preferred embodiment, the other active ingredient is melphalan. In another preferred embodiment, the other active ingredient is bortezomib. In another preferred embodiment, the other active ingredient is lenalidomide or thalidomide.

In another preferred embodiment of this second aspect of the invention, the monoclonal gammopathy is selected from multiple myeloma, plasma cell leukemia, Waldenstrom's macroglobulinemia, amyloidosis, or any of the combinations thereof. In a more preferred embodiment, the monoclonal gammopathy is multiple myeloma.

The pharmaceutically acceptable adjuvants and vehicles which can be used in said compositions are adjuvants and vehicles known by those skilled in the art and commonly used in the production of therapeutic compositions.

In the sense used herein, the expression "therapeutically effective amount" refers to the amount of the agent or compound capable of developing the therapeutic action determined by the pharmacological properties thereof, which is calculated to produce the desired effect, and will generally be determined, among other causes, by the actual characteristics of the compounds, including the patient's age, condition, the severity of the abnormality or disorder, and the route and frequency of administration.

The compounds described in the present invention, the salts, prodrugs, and/or solvates thereof, as well as the pharmaceutical compositions containing them, can be used together with other additional drugs or active ingredients to provide a combination therapy. Said additional drugs can be part of the same pharmaceutical composition, or alternatively can be provided in the form of a separate composition for simultaneous or non-simultaneous administration with the pharmaceutical composition comprising a compound of formula (I), or a salt, prodrug, or solvate thereof.

As it is used herein, the term "active ingredient," "active substance," " pharmaceutically active substance ", or "pharmaceutically active ingredient" means any component that potentially provides pharmacological activity or another different effect in the diagnosis, cure, mitigation, treatment, or prevention of a disease, or that affects the structure or function of the body of humans or other animals. The term includes those components that promote a chemical change in the production of the drug and are present therein in a modified form envisaged for providing the specific activity or effect.

Another aspect of the invention relates to a dosage form, hereinafter dosage form of the invention, comprising the compound of the invention or the composition of the invention.

In this specification, "dosage form" is understood as the mixture of one or more active ingredients with or without additives having physical characteristics for suitable dosing, preservation, administration, and bioavailability.

In another preferred embodiment of the present invention, the pharmaceutical compositions and dosage forms of the invention are suitable for oral administration in solid or liquid form. The possible forms for oral administration are tablets, capsules, syrups, or solutions and they may contain conventional excipients known in the pharmaceutical field, such as binding agents (e.g., syrup, acacia, gelatin, sorbitol, tragacanth, or polyvinylpyrrolidone), fillers (e.g., lactose, sugar, cornstarch, calcium phosphate, sorbitol, or glycine), disintegrants (e.g., starch, polyvinylpyrrolidone, or microcrystalline cellulose) or a pharmaceutically acceptable surfactant such as sodium lauryl sulfate. Other dosage forms can be colloidal systems which include, among others, nanoemulsions, nanocapsules, and polymer nanoparticles.

The compositions for oral administration can be prepared as a mixture and dispersion using conventional Galenic pharmacy methods. The tablets can be coated following the methods known in the pharmaceutical industry.

The pharmaceutical compositions and dosage forms can be adapted as sterile solutions, suspensions, or lyophilisates of the products of the invention for parenteral administration using the suitable dose. Suitable excipients, such as pH-buffering agents or surfactants, can be used.

The formulations mentioned above can be prepared using conventional methods, such as those described in the pharmacopoeias of different countries and in other reference texts.

As it is used herein, the term "medicinal product" refers to any substance used for the prevention, diagnosis, alleviation, treatment, or curing of diseases in humans and animals.

The administration of the compounds, pharmaceutical compositions, or dosage forms of the present invention can be performed by means of any suitable method, such as intravenous infusion and through the oral, topical, or parenteral routes. Oral administration is preferred given the convenience it offers to the patients and given the chronic character of the diseases to be treated.

The administered amount of a compound of the present invention will depend on the relative efficacy of the chosen compound, the severity of the disease to be treated, and the patient's weight. However, the compounds of this invention will be administered one or more times a day, for example 1, 2, 3, or 4 times a day, with a total dosage between 0.1 and 1000 mg/kg/day. It is important to take into account that variations in the dose, as well as modifications in the route of administration, may have to be introduced depending on the patient's age and condition.

The compounds and compositions of the present invention can be used together with other medicinal products in combined therapies. The other drugs can be part of the same composition or of another different composition, for administration at the same time or at different times.

### COMBINED PREPARATION OF THE INVENTION AND USES

A **third aspect** of the invention relates to a combined preparation, hereinafter combined preparation of the invention, comprising or consisting of:
a) component A which is a compound (compound of the invention) or a composition (composition of the invention) as defined in the present invention, and
b) component B which is an active ingredient selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof.

In an even more preferred embodiment, the active ingredient of (b) is dexamethasone. In another even more preferred embodiment, the active ingredient of (b) is melphalan. In another preferred embodiment, the other active ingredient is bortezomib. In another preferred embodiment, the other active ingredient is lenalidomide or thalidomide.

In another preferred embodiment, the combined preparation of the invention further comprises pharmaceutically acceptable excipients. In another preferred embodiment, the combined preparation of the invention comprises, as active ingredients, only those mentioned above, although it may comprise other pharmaceutically acceptable excipients and vehicles.

A **fourth aspect** relates to the use of the combined preparation of the invention, wherein components (a) and (b) are administered simultaneously, separately, or sequentially for the prevention, relief, improvement, and/or treatment of a disease. Alternatively, it relates to the combined preparation of the invention for simultaneous, separate, or sequential use in therapy.

A preferred embodiment of this aspect relates to the use of the combined preparation of the invention in the production of a medicinal product for simultaneous, separate, or sequential use in the treatment of cancer. Alternatively, it relates to the combined preparation of the invention for simultaneous, separate, or sequential use for the treatment of cancer.

In a preferred embodiment of this aspect of the invention, the cancer is a hematological cancer. More preferably, the hematological cancer is acute myeloid leukemia or monoclonal gammopathy. In a more preferred embodiment of this aspect of the invention, the cancer is acute myeloid leukemia. In another more preferred embodiment of this aspect of the invention, the cancer is a monoclonal gammopathy.

In another preferred embodiment of this aspect, the monoclonal gammopathy is selected from multiple myeloma, plasma cell leukemia, Waldenstrom's macroglobulinemia, amyloidosis, or any of the combinations thereof. In a more preferred embodiment, the monoclonal gammopathy is multiple myeloma.

As it is understood herein, the term "treatment" refers to fighting the effects resulting from a disease or pathological condition of interest in a subject (preferably mammal, and more preferably a human) which includes:
(i) inhibiting the disease or pathological condition, i.e., stopping its development;
(ii) alleviating the disease or pathological condition, i.e., causing the regression of the disease or pathological condition or its symptomatology;
(iii) stabilizing the disease or pathological condition.

As it is understood herein, the term "prevention" consists of preventing the onset of the disease, i.e., preventing the disease or pathological condition from occurring in a subject (preferably mammal, and more preferably a human), particularly when said subject has a predisposition to the pathological condition.

The compounds of the invention can be in a crystalline form as free compounds or solvates, and both forms are expected to fall within the scope of the present invention. Solvation methods are generally known in the art. Suitable solvates are pharmaceutically acceptable solvates. In a particular embodiment, the solvate is a hydrate.

The compounds of the invention or the salts or solvates thereof are preferably in a pharmaceutically acceptable form or a substantially pure form. Pharmaceutically acceptable form is understood, *inter alia,* as having a pharmaceutically acceptable level of purity, excluding normal pharmaceutical additives such as diluents and excipients, and without including any material considered toxic at normal dosage levels. The levels of purity for the compound of the invention are preferably above 50%, more preferably above 70%, and even more preferably above 90%.

In a preferred embodiment, it is above 95% of the compound of the invention or of the salts, solvates, or prodrugs thereof.

The compounds of the present invention may include enantiomers depending on the presence of chiral centers, or isomers depending on the presence of multiple bonds (for example, Z, E). The individual isomers, enantiomers, or diastereomers and mixtures thereof fall within the scope of the present invention. A compound drawn with explicit stereochemistry is for the purpose of depicting the racemic structure with the relative stereochemistry, as well as the enantiomers in different degrees of purity. In any case, the enantiomers and diastereoisomers of the compounds that are depicted with a particular stereochemistry are also part of the compounds of the invention.

Said compositions can have one or more indazole agents. Said indazole agents may be combined in the same or different proportions, and may be part of the same formulation, or may be formulated in different formulations for sequential, joint, or simultaneous administration.

The pharmaceutical compositions of the invention are administered topically, transdermally, orally, nasally, intramuscularly, intravenously, intraperitoneally, subcutaneously, enterally, or parenterally. Illustrative examples of topical or transdermal administration include, but are not limited to, iontophoresis, sonophoresis, electroporation, mechanical pressure, osmotic pressure gradient, occlusive cure, microinjections, needle-free injections by means of pressure, microelectric patches, and any combination thereof. Illustrative examples of dosage forms for oral administration include pills, capsules, pellets, solutions, suspensions, etc., and may contain conventional excipients, such as binders, diluents, disintegrants, lubricants, humectants, etc., and can be prepared using conventional methods. The pharmaceutical compositions can also be adapted for parenteral administration in the form of, for example, sterile lyophilized solutions, suspensions, or products, in the suitable dosage form; in this case, said pharmaceutical compositions will include suitable excipients, such as buffers, surface active agents, etc. In any case, the excipients will be chosen depending on the selected pharmaceutical dosage form. A review of the different pharmaceutical dosage forms of drugs and the preparation thereof can be found in the book entitled "Tratado de Farmacia Galenica," by C. Faulí i Trillo, 10th edition, 1993, Luzán 5, S.A. de Ediciones.

Both the compositions of the present invention and the combined preparation can be formulated for administration to an animal, and more preferably to a mammal, including humans, in a variety of forms known in the state of the art. In that sense, they can be, without limitation, in sterile aqueous solution or biological fluids, such as serum. The aqueous solutions may or may not be buffered and have additional active or inactive components. The additional components include ionic force-modulating salts, preservatives including, but without limitation, antimicrobial agents, antioxidants, chelating agents, and the like, and nutrients including glucose, dextrose, vitamins, and minerals. Alternatively, the compositions can be prepared for administration in solid forma. The compositions can be combined with several inert vehicles or excipients, including, but without limitation, binders such as microcrystalline cellulose, tragacanth gum, or gelatin; excipients such as starch or lactose; dispersing agents such as alginic acid or cornstarch; lubricants such as magnesium stearate, glidants such as colloidal silicon dioxide; sweetening agents such as sucrose or saccharine; or aromatizing agents such as mint or methyl salicylate.

Such compositions or combined preparations and/or the formulations thereof can be administered to an animal, including a mammal, and therefore humans, in a variety of forms including, but without limitation, intraperitoneally, intravenously, intramuscularly, subcutaneously, intrathecally, intraventricularly, orally, enterally, parenterally, intranasally, or dermally.

The dosage for obtaining a therapeutically effective amount depends on a variety of factors, such as the age, weight, sex, or tolerance of the mammal, for example. In the sense used herein, the expression "therapeutically effective amount" refers to the amount of indazole agent or agents which produce the desired effect, and will generally be determined, among other causes, by the actual characteristics of said prodrugs, derivatives, or analogs and the therapeutic effect to be achieved. The "adjuvants" and "pharmaceutically acceptable vehicles" which can be used in said compositions are vehicles known by those skilled in the art.

It must be stressed that the term "combined preparation" or also "juxtaposition" in this specification means that the components of the combined preparation do not have to be present as a combination, for example in a composition, to be available for the separate or sequential application thereof. Therefore, the expression "juxtaposed" means that it is not necessarily a real combination in view of the physical separation of the components.

Another aspect relates to a method for the treatment of a monoclonal gammopathy, comprising the administration of a compound of the invention or any of the pharmaceutically acceptable salts, esters, tautomers, solvates, and hydrates thereof, or any of the combinations thereof, as defined above.

In a preferred embodiment of this aspect of the invention, the composition further comprises one or more pharmaceutically acceptable excipients or vehicles. Preferably, the composition of the invention is a pharmaceutical composition comprising a compound of the invention as the only active ingredient, although it may comprise one or more pharmaceutically acceptable excipients and/or vehicles. In another preferred embodiment, the composition further comprises another active ingredient. In a more preferred embodiment, the other active ingredient is selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof. In an even more preferred embodiment, the other active ingredient is dexamethasone. In another even more preferred embodiment, the other active ingredient is melphalan. In another preferred embodiment, the other active ingredient is bortezomib. In another preferred embodiment, the other active ingredient is lenalidomide or thalidomide.

In another preferred embodiment of this second aspect of the invention, the monoclonal gammopathy is selected from multiple myeloma, plasma cell leukemia, Waldenstrom's macroglobulinemia, amyloidosis, or any of the combinations thereof. In a more preferred embodiment, the monoclonal gammopathy is multiple myeloma.

### METHOD FOR PREPARING THE COMPOUNDS OF THE INVENTION

The preparation of indazole ether derivatives to be used according to the invention is described in European Journal of Medicinal Chemistry 2014, 73, 56-72 (EJMC-2014) and in patent PCT/ES2010/000400.

The compounds were prepared in several steps according to the methods described in EJMC-2014. The first step consists of protecting the nitrogen in position 1 of the indazole derivatives by means of ethyl chloroformate reaction. The second step consists of introducing the R₂ group. The third step consists of deprotecting the nitrogen in position 1 and introducing the substituent R₃ by means of reaction with the corresponding halides, where R₁, R₂, and R₃ have the aforementioned meaning.

In the patent (PCT/ES2010/000400), these indazole derivatives are claimed for the treatment, prevention, or improvement of glaucoma, bronchial asthma, and chronic bronchitis, allergies such as contact dermatitis or allergy conjunctivitis, arthritis, pain, diseases associated with organ transplants, motor disorders associated with Tourette syndrome, Parkinson's disease, or Huntington's chorea, multiple sclerosis, emesis, and other toxic or undesirable effects associated with anti-cancer chemotherapy and appetite therapy.

The article (EJMC-2014) describes these indazole ether derivatives as potential drugs for the treatment of Alzheimer's disease.

Throughout the description and claims, the word "comprises" and variants thereof do not seek to exclude other technical features, additives, components, or steps. For those skilled in the art, other objects, advantages, and features of the invention will be inferred in part from the description and in part from putting the invention into practice. The following examples and drawings are provided by way of illustration and do not seek to be limiting of the present invention.

### EXAMPLES OF THE INVENTION

### MULTIPLE MYELOMA

The examples show that the compounds of the invention exert a pro-apoptotic effect on MM cells, without affecting the viability of healthy cells, by selectively interacting with CB2 receptors, triggering pro-apoptotic activity through the caspase-2 pathway, increasing pro-apoptotic regulators and reducing anti-apoptotic regulators, increasing the *de novo* synthesis of ceramide, and reducing mitochondrial membrane potential.

Furthermore, these new compounds inhibit tumor growth *in vivo,* and increase susceptibility to anti-myeloma drugs such as dexamethasone and melphalan.

Therefore, this invention represents a very promising therapy for multiple myeloma and related diseases.

### Example 1. Materials and methods

### Statement of Ethics

Any research that involves animal or human samples was approved by the Clinical Research Ethics Committee (*Comité Ético de Investigación Clínica*-CEIC) of Hospital Universitario Virgen del Rocío, and was carried out in accordance with the Declaration of Helsinki.

### Cell cultures of multiple myeloma and patient cells

*In vitro* studies were carried out using six different human MM cell lines, U266, RPMI8226, MM1S, MM1R, U266-LR7, and RPMI-LR5. For *ex vivo* assays, human primary cells from healthy donors and MM patients were used (Table 1). Human MM cell lines U266, RPMI8226, and MM1.S were acquired from ATCC and lines U266-LR7, RPMI-LR5, and MM1.R were kindly provided by Dr. Enrique Ocio (Hospital Universitario de Salamanca, Spain). The primary cells were obtained from bone marrow (BM) aspirates or peripheral blood (PB) samples, and the peripheral blood mononuclear cells (PBMCs) were isolated by Ficoll-Hypaque centrifugation and washed twice in phosphate buffered saline (PBS) containing 1% BSA. Hematopoietic stem cells and B and T lymphocytes were isolated from healthy PB donors by positive immunomagnetic separation using human MACS CD34+, CD19+, and CD3+ microbeads, respectively. MM plasma cells were obtained from the bone marrow (BM) of patients with an infiltration of cells of more than 30% (Table 1). The MM plasma cells were identified using CD138+, and were then distinguished from the other cell populations by flow cytometry using a suitable combination of antibodies: anti-human CD64-FITC, CD34-PE, CD56-APC, CD38-APC-H7, and CD45-Pacific Blue antibodies (BD Biosciences, San Jose, CA). All the cell lines were cultured in RPMI-1640 supplemented with 10% fetal bovine serum (FBS) and 1% penicillin/streptomycin, as recommended by the supplier. For human primary cells, the concentration of FBS was up to 20%.

**Table 1. Clinical characteristics of patients from whom primary myeloma plasma cells were obtained. UPN: unique patient number; del = deletion; t = translocation; amp = amplification.**

| PATIENT ID | CYTOGENETICS | RESPONSE in the first line |
|---|---|---|
| UPN-1 | Translocated IgH (14q32) | Primary refractory |
| UPN-2 | t(4:14) | Partial response |
| UPN-3 | del(17p)p53 | Primary refractory |
| UPN-4 | t(11:14) | Death in diagnosis |
| UPN-5 | del(13q14), del(17p)p53, hypodiploidy | Partial response |
| UPN-6 | amp(1q), trisomy del 7 | Refractory disease |

### Medicinal products and treatments

Cannabinoid agonists WIN-55,212-2 mesylate were acquired from Tocris Bioscience. Indazole agonists PGN-6, -17, -34, and -72, and selective CB2 antagonists PGN-8, -37, and -70 were synthesized and kindly provided by Dr. Nuria Campillo of the Centro de Investigaciones Biologicas, Madrid. Fumonisin B1 (FB1) was obtained from Enzo Life Sciences, Z-VAD (OMe)-FMK (pan-caspase inhibitor) was obtained from Abcam, and TMRE (tetramethylrhodamine methyl ester perchlorate) was obtained from Santa Cruz Biotechnology (Santa Cruz, CA). The anti-myeloma agents, dexamethasone and melphalan, were provided by the pharmacy department of Hospital Universitario Virgen del Rocío.

### Western blot and antibodies

The extracts for Western blot were taken after 0, 2, 6, 18, and 24 hours. The cells were lysed according to Gilbert et al., 2002. (J Immunol Methods. 2002, 271:185-201), by adding 2% ASB-14 (Calbiochem, Beeston, United Kingdom) to the isotonic lysis buffer. The protein concentration was determined by Pierce® Microplate BCA Protein Assay kit-Reducing Agent Compatible (Pierce, Rockford, IL). The samples were subjected to SDS/PAGE in AnykD precast gels (Bio Rad, Hercules, CA) and transferred to PVDF membranes using Trans-Blot® TurboTM System (Bio-Rad). The membranes were incubated over night at 4°C with primary antibody in 0.05% Tween 20-Tris buffered saline (TTBS) then with the suitable secondary antibody, and they were subjected to chemiluminescence detection. As a control condition, the cells were treated with DMSO (<0.15%) in RPMI 1640 medium from Gibco (Gaithersburg, MD).

The antibodies for caspase-2, -8, -9, active caspase-3, -p-Akt (phospho T308), p-Erk (T202 + Y204), -p-p38MAPK (phospho T180 + Y182), -p-JNK (phospho T183 + Y185), -SPT, and the CB1 and CB2 receptors were from Abcam, anti-MCL-1 and -Bcl-xL were from Santa Cruz Biotechnology, anti-PARP was from Cell Signaling Technology, anti-Bax and -Bak were from BD Biosciences. Anti-beta-tubulin was from Sigma-Aldrich. All the horseradish peroxidase (HRP)-conjugated secondary antibodies used were from Jackson ImmunoResearch and produced in a donkey to prevent the possible cross-reactivity when several tests were performed.

### Cell viability analysis

The MM cell lines and primary cells were exposed to different doses of indazole compounds, and viability was evaluated at 18, 48, and 72 hours. Pretreatments with the ceramide synthesis inhibitor (FB1), pan-caspase (ZVAD-FMK), and CB antagonists (PGN-8, PGN-37, and PGN-70) were performed for 30 minutes, and then the cells were incubated with indazole compound WIN-55 up to 18 hours. Cell viability was determined using the MTT assay Cell Counting Kit-8 (Dojindo, Kumamoto, Japan) according to the manufacturer's instructions.

The cell viability of the MM cell lines and primary cells from healthy PB donors was also evaluated by flow cytometry using 7-AAD/Annexin V. Patient bone marrow (BM) cells were analyzed using 7-AAD with a combination of monoclonal antibodies against myeloma-associated antigens (anti-CD56-APC, anti-CD45-Pacific Blue, and anti-CD38-APC-H7 [BD Biosciences]) and antibodies for distinguishing the granulomonocytic population (anti-CD64-FITC) and lymphocytic population (anti-CD45-Pacific Blue). The cells were acquired by means of FACSCanto II flow cytometer (BD Biosciences) and analyzed using InfinicytTM Software (Cytognos, Spain).

The evaluation of the synergy of the indazole compound WIN-55 with other anti-myeloma agents, such as dexamethasone and melphalan, was done by evaluating cell viability by means of an MTT assay. The strength of the combination was quantified with Calcusyn software (Biosoft, Ferguson, MO), which is based on the Chou Talalay method, and it calculates a combination index (CI) with the following interpretation: CI>1: antagonist effect, CI=1: additive effect and CI<1: synergistic effect.

### Mitochondrial transmembrane potential analysis

Cell lines U266 were exposed to 50 µM of WIN-55 for 15, 30, 45, and 60 minutes. The loss of mitochondrial membrane potential (Δψm) was calculated using TRME (tetramethylrhodamine-ethyl-ester-perchlorate) according to the manufacturer's instructions and CCCP (2-[2-(3-chlorophenyl)hydrazinylidene] propanedinitrile) was used as a control to induce the loss of Δψm.

### Immunofluorescence

The cells treated for 6 hours with indazole compounds were collected and placed on a slide. Immunofluorescence staining was performed as described previously by Vielhaber et al. 2001 (Glycobiology. 2001,11:451-7) using anti-ceramide as the primary antibody. The ceramide antibody was obtained from Sigma-Aldrich, and the Alexa-488-conjugated secondary antibody was obtained from Abcam. As a control condition, the cells were treated with DMSO (<0.15%) in RPMI 1640 medium from Gibco (Gaithersburg, MD).

### MM xenografts

The "NOD/scid/IL-2R gammae null" (NGS) mice were acquired from Charles River (France). The tumor xenografts were induced by the subcutaneous injection of 5×10⁶ U266 cells mixed with 100 µl of Matrigel (BD Biosciences) in 8-week old mice. When the tumors became palpable (> 0.5 cm), the mice were randomly assigned in the following groups (10 mice per group), which received i.p.: 1) 5 mg/kg WIN-55 every 24 hours, 2) 5 mg/kg WIN-55 every 48 hours, and 3) a vehicle. Two groups were left tumor-free and served as a negative control, receiving treatment every 24 or 48 hours, respectively. The tumor growth was evaluated daily by measuring the two bisecting diameters of the tumor with a digital Vernier gauge or caliper. The volume was calculated using the following formula: volume= length x (width) e2 x 0.4 mm³. The animals were sacrificed when the length or width of the tumor reached 2 cm.

### Statistics

Unless otherwise indicated, an experiment representative of at least three independent experiments is shown. For all the statistical analyses, the data was analyzed by means of Student's T-test with the SPSS software with a statistical significance P ≤ 0.05. The means and standard deviations were also determined. The synergy was calculated using R as described in the Calcusyn software; a combination index (CI) <1 indicates synergy, and >1 indicates antagonism.

### Example 2. The indazole compounds of the invention have a highly selective antiproliferative effect on MM cells

First, the effect of different indazole compounds of the invention on the proliferation and cell viability of several MM cell lines was evaluated by means of the MTT assay and flow cytometry. It was found that incubation with WIN-55, which is a non-selective indazole compound, significantly reduced the cell viability of all the MM cell lines compared with untreated control cells at 18 hours (Figure 1). The sensitivity pattern shown in the MTT assay (Figure 1A and Table 2) ranged from U266, this being the most resistant cell line (IC50 = 17.24 µM), to RPMI, which was the most sensitive one (IC50=11.66 µM). It also confirmed the reduction in cell viability by flow cytometry for the two cell lines mentioned above (Figure 1B), and IC50 values similar to those obtained by means of the MTT assay for U266 (IC50 = 18.51) and RPMI (IC50 = 12.66) were obtained. The cell viability analysis by means of the MTT assay and flow cytometry for longer times, i.e., 48 and 72 hours, also showed similar results (Figure 1D).

Furthermore, all the MM cell lines were treated with six different indazole compounds, PGN-6, -17, -34, and -72, which were characterized by their higher selectivity for the CB2 receptor compared with WIN-55. As shown in Figure 1C, these compounds of the PGN family induced a variable but significant antiproliferative effect on the different MM cell lines (Table 2).

The effect of the indazole compounds was more broadly examined *ex vivo* in myeloma plasma cells (MPCs) of six MM patients by flow cytometry using WIN-55. After treatment, the MPCs (CD38+) showed a significant decrease in cell viability from 70 to 85% at 20 and 50 µM, respectively (Figure 2A). In contrast, the cell viability of the analyzed normal cell subpopulations, including granulomonocytes (CD64+) and lymphocytes (CD45+), obtained from patient bone marrow, was barely affected. Only the highest dose tested, i.e., 50 µM, induced an antiproliferative effect on the lymphocyte population (CD45+). For this reason, the two main lymphocyte populations, the B-cells (CD19+) and the T-cells (CD3+) obtained from healthy individuals, were isolated, and then their viability was analyzed separately. As shown in Figure 2B, the antiproliferative effect observed in the lymphocyte population was primarily due to the effect on the B-cells (CD19+). Furthermore, the effect of WIN-55 on the hematopoietic stem cells (CD34+) from healthy donors was tested, and, surprisingly, hematopoietic stem cell viability was not affected by treatment with the indazole compound/compounds of the invention (Figure 2B). Finally, the effect of two of the indazole compounds PNG, PGN-6 and PGN-17, was also evaluated. These compounds did not affect the viability of the lymphocytes at the doses at which a significant antiproliferative effect on MM cell lines was observed.

These results indicate that the indazole compounds of the invention have a very selective pro-apoptotic effect on the myelomatous cells, whereas the viability of the healthy cells, including the hematopoietic precursor cells, is not affected.

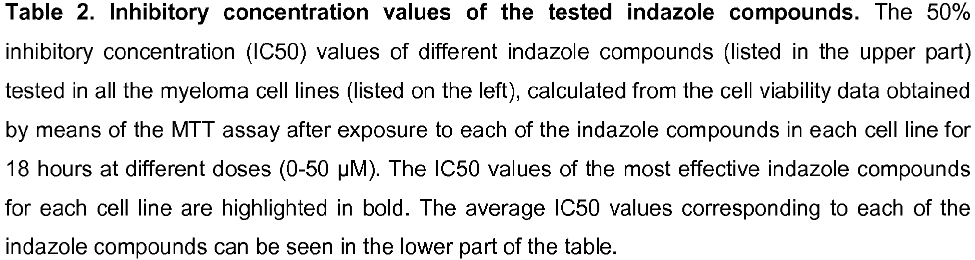

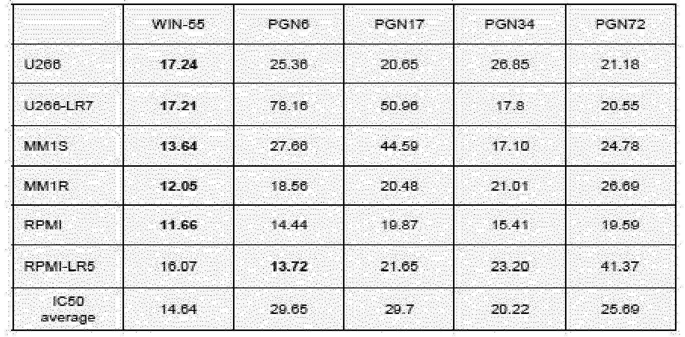

### Example 3. The effect of the indazole compounds of the invention is mediated by apoptotic mechanisms

In order to evaluate if the antiproliferative effect of WIN-55 is mediated by apoptotic mechanisms, the expression of PARP, caspases, and other pro/anti-apoptotic proteins (Bcl-2 family) in the most resistant MM cell line, U266, was analyzed. Treatment with the compounds of the invention induced a decrease in the expression of the full form of PARP in a time-dependent manner, with a concomitant increase in the expression of the 89 kDa fragment (CL_85kDa), which could be detected 2 hours after exposure (Figure 3A). Furthermore, caspase-3 activation was evaluated using an antibody that recognizes its cleaved forms of 17 kDa (CL_17kDa) and 12 kDa (CL_12kDa), respectively. The expression of both cleaved forms increased over time, simultaneously with PARP fragmentation. This indicated that the antiproliferative effect of WIN-55 was consistent with an induction of caspase-3 activation. For the purpose of knowing which of the main pathways of apoptosis (extrinsic, intrinsic, or associated with endoplasmic reticulum stress) was activated by the indazole compound, the expression of the main initiator caspases, caspase-9 (Casp-9), -8 (Casp-8), and -2 (Casp-2), respectively, was evaluated. As shown in Figure 3A, the expression of the three pro-caspases (PRO) decreased after 2 hours of exposure to the indazole compounds of the invention, but only in the case of Casp-2 was a strong increase detected in the expression of all the cleaved forms over time (CL_32 kDa, CL_18 kDa, CL_14 kDa). Therefore, Casp-2 processing and activation were much more considerable than what was observed for Casp-8 and -9. For the purpose of elucidating the mechanisms involved in the apoptosis induced by the compounds of the invention, several proteins of the Bcl-2 family, such as Mcl-1, Bcl-xL, Bax, and Bak involved in the apoptosis process, were then analyzed. Western blot analysis (Figure 3B) showed a considerable increase in the pro-apoptotic regulators Bak and Bax, whereas the expression levels of the anti-apoptotic proteins Bcl-xL and Mcl-1 decreased over time. The pro-apoptotic effect of WIN-55 was also confirmed as being mediated by caspase activation by incubating the cells treated with WIN-55 with/without the pan-caspase inhibitor Z-VAD-FMK. As shown in Figure 3C, the pan-caspase inhibitor prevented the apoptosis induced by compounds of the invention in both the most resistant and the most sensitive cell lines, i.e., U266 and RPMI, respectively, when were they are co-treated at suboptimal concentrations, i.e., below their respective IC50 values (Figure 3C). These results show that the effect of the compounds of the invention on MM cells is mediated, at least in part, by apoptotic mechanisms, with the Casp-2 pathway being the most strongly activated pathway.

### Example 4. Target signaling pathways of the compounds of the invention in myelomatous cells

For the purpose of exploring which signaling pathways are mainly involved in the apoptosis induced by compounds of the invention, various parameters were evaluated:

### Expression profile of phosphorylated JNK-, Erk1/2-, p38-MAPK-, and Akt-

Treatment with the compounds of the invention slightly overexpressed p-JNK and p-ERK1/2, whereas it moderately reduced the expression of p-p38-MAPK over incubation time. Oddly enough, WIN-55 induced a significant overexpression of p-Akt (phospho-T308) at early points in time, whereas at later times expression levels showed a decrease (Figure 3D). According to these results, WIN-55 slightly modulates different signaling pathways involved in the balance between survival/death; however, the Akt pathway is strongly modulated and shows a biphasic response, with a short-time activation and long-term down-regulation.

### De novo synthesis of ceramides

The *de novo* synthesis of ceramides is involved in the apoptosis induced by the compounds of the invention. Therefore, the expression of ceramides was evaluated by means of immunofluorescence in MM cells exposed to WIN-55, and a considerable increase in the expression of ceramides in U266 cells treated with the compound of the invention compared with untreated cells (Figure 4A) was detected. Furthermore, the expression level of serine-palmitoyltransferase (SPT), which is the enzyme that limits the speed in the *de novo* synthesis of ceramide, increased in U266 cells after incubation with WIN-55 (Figure 4B). A moderate increase in SPT was observed 2 hours after treatment, which reached its maximum level at 18 hours. To confirm the involvement of this phospholipid in the apoptosis induced by the compound of the invention, the MM cells were preincubated with fumonisin B1 (FB1), a ceramide synthesis inhibitor. As shown in Figure 4C, the pharmacological blockade of ceramide synthesis considerably prevented the PARP fragmentation induced by WIN-55 in U266 cells. Furthermore, FB1 significantly reverses the effect induced by the indazole compound in both cell lines U266 and RPMI (the most resistant and the most sensitive cell lines, respectively), as was evaluated by means of MTT assays (Figure 4D). This data confirmed that ceramide plays a crucial role in the apoptosis induced by compounds of the invention in MM cells.

### Response to endoplasmic reticulum stress and Δψm

The tested compound of the invention attenuates the response to endoplasmic reticulum stress in myelomatous cells and induces an early loss of mitochondrial membrane potential. Given that myelomatous cells have a highly developed endoplasmic reticulum, they are prone to said organelle suffering stress. As a result, the effect of WIN-55 on the expression of certain endoplasmic reticulum stress marker proteins in U226 cells is evaluated. In contrast to what was expected, there was a slight but sustained decrease in the expression of endoplasmic reticulum stress marker proteins CHOP, ATF-4, p-IRE1, and XBP-1s ("spliced form") compared with the control, and a slight increase in the level of XBP-1u ("unspliced" form), which indicates the lack of reticulum stress saturation, in cells treated with the compound/compounds of the invention compared with the control cells. This observation suggests that the tested compound of the invention reduces, or at least does not overload/saturate, the Unfolded Protein Response (UPR), which is activated under conditions of endoplasmatic reticulum stress in MM cells (Figure 5A).

It is important to point out that the loss of mitochondrial membrane potential is a point of no return in apoptosis. As a result, changes in Δψm in MM cells treated with the compound/compounds of the invention were analyzed. A considerable drop in Δψm in U266 cells after 15 minutes of incubation was observed, and it continued to slightly drop over time (Figure 5B).

### Example 5. The effect of the compounds of the invention on MM cells is mediated by CB2 receptors

In order to know if the pro-apoptotic effect of WIN-55 is mediated through the CB2 receptor, the effect of WIN-55 after treatment with three different selective CB2 antagonists, i.e., PGN-8, -37, and -70, was evaluated. As shown in Figure 5C, the preincubation of cells with all the tested CB2 antagonists (PGN-8, PGN-37, and PGN-70) considerably inhibited the pro-apoptotic effect induced by WIN-55 in both the most resistant and the most sensitive cell lines, i.e., U266 and RPMI, respectively. These results confirm that the effect of WIN-55 is mediated by CB2.

The expression of CB2 in different MM cell lines, as well as in normal hematopoietic cells from healthy individuals, was then evaluated. CB2 partially shows a 40 kDa band, consistent with the weight of the CB2 monomer, and another 30 kDa band which corresponds to the truncated form (Figure 5D). The strong expression level of the 40 kDa band was detected in the cell lines most sensitive to WIN-55, MM1R and RPMI, as well as in the lymphocytes (LB and LT), whereas it was virtually non-immunoreactive in the most resistant cell lines, U266 and MM1S, and in the hematopoietic stem cells (CD34+). In contrast, the highest expression levels of the truncated CB2 receptor were observed in the most sensitive cell line, i.e., RPMI, and in B-cells (LB).

### Example 6. WIN-55 enhances the efficacy of anti-myeloma agents

Anti-myeloma therapies consist of combinations of drugs with different mechanisms of action. For this reason, the effect of the compound of the invention WIN-55 on a dual combination with dexamethasone and melphalan, not only in cell lines U266 and RPMI, but also in their corresponding cell lines resistant to melphalan, U266-LR7 and RPMI-LR5, was analyzed. The combination index (CI) obtained from the analysis of cell viability data indicates that WIN-55 had a synergistic effect with both dexamethasone (DEX) and melphalan (MPH) (see Figure 6). The combination dose of WIN-55 was suboptimal according to the IC50 for each cell line, i.e., 20 µM for U266, U266-LR7, and RPMI-LR5, and 10 µM for RPMI. In all cases, the combination with dexamethasone or melphalan resulted in a synergistic response, even in melphalan-resistant cell lines U266-LR7 and RPMI-LR5. The results of the study indicate that the compound of the invention WIN-55 in combination with dexamethasone or melphalan, acts not only synergistically, but it also overcomes resistance in MM cell lines.

### Example 7. The administration of indazole compounds inhibits tumor growth in vivo

Finally, the antitumor effect of the compounds of the invention was analyzed *in vivo,* using a human MM xenograft model in immunodeficient NOD/SCID (NSG) mice. The MM cell line that is most resistant *in vitro* was used for this purpose. A considerable and progressive loss of tumor volume was observed after the administration of compounds of the invention compared with the corresponding ones treated with a vehicle (Figure 7).

### ACUTE MYELOID LEUKEMIA

### Materials and methods

### Cell cultures

The cell lines of acute myeloid leukemia KG-1a and HL60 (American Type Culture Collection) were cultured in IMDM medium supplemented with 2 mM of L-glutamine, 100 mg/mL of penicillin, 100 µg/ml of streptomycin, and 15% fetal bovine serum (FBS).

U937, NB-4, KG-1, and MOLM-13 (DMSZ, Braunschweig, Germany) were cultured in complete RPMI 1640 medium (with 2 mM of L-glutamine, 100 mg of penicillin, 100 µg/ml of streptomycin, and with 10% FBS), supplemented with 10 mM HEPES, 1x NaPuvuvato, and 1x non-essential amino acids.

All the cells were cultured in a humidified atmosphere of CO₂/air (5%/95%).

The human primary cells were obtained from the bone marrow (BM) of AML patients and peripheral blood (PB) of healthy donors. Hematopoietic progenitor cells (CD34+) were isolated from samples of leukapharesis, and lymphocytes B (CD19+) and T (CD3+) from the buffy coats by means of positive immunomagnetic separation in the professional AutoMACS separator (Miltenyi Biotec, Bergisch Gladbach, Germany) according to the manufacturer's instructions .

This study was approved by the Clinical Research Ethics Committee *(Comité Ético de Investigación Clínica*- CEIC) of Hospital Universitario Virgen del Rocío, and informed consents were obtained from all the patients and donors in accordance with the Declaration of Helsinki.

### PGN family cannabinoid synthesis

Cannabinoids PGN-6, -17, -34, -128, and -153, and selective CB2 antagonists PGN-8, -37, and -70 were synthesized in Dr. Páez's laboratory at the Centro de Investigaciones Biologicas, Madrid. PGN-6, -17, and -34 were synthesized following the method published by Gonzalez-Naranjo et al. 2014. Eur J Med Chem 73, 56-72.

Cannabinoids PGN-43, -128, and -153 were synthesized following the method published in patent document PCT/ES2016/070906.

### Compounds and treatments

WIN-55,212-2((R)-(+)-[2,3-dihydro-5-methyl-3-(4-morpholinomethyl)-pyrrolo[1,2,3]-1,4-benzoxazin-6-yl]-1-naphthalenylmethanone mesylate were acquired from Tocris Bioscience (Bristol, UK), and cannabinoid agonists PGN6, PGN17, PGN43, PGN34, PGN128, and PGN153 and selective CB2 antagonists PGN-8, -37, and - 70 were synthesized in Dr. Páez's laboratory at the Centro de Investigaciones Biológicas of Madrid.

They were added at the indicated concentrations to the culture medium for different incubation periods. The control cells were cultured with the relevant amounts of DMSO.

Myriocin (ISP-1), the serine-palmitoyltransferase (SPT) enzyme inhibitor, was obtained from Enzo Life Sciences (Lausen, Switzerland) and Z-VAD (OMe)-FMK (caspase inhibitor) was obtained from Abcam.

Cytarabine was supplied by the Pharmacy Department of the Hospital Universitario Virgen del Rocío.

### Cell viability and apoptosis assays

The cell lines and primary cells were cultured in 96-well plates (5 x 10e5 cells per well) with the addition of the indicated concentrations of WIN 55,212-2 or PGN cannabinoids in DMSO or with the solvent only in triplicate at 18, 48, and 72 hours. Caspase inhibitor Z-VAD (OMe)-FMK or the CB2 receptor antagonist PGN-8, -37, and -70, together with the cannabinoid WIN-55,212-2, were also added. Cell viability was determined by means of the WST-1 assay [2-(4-iodophenyl)-3-(4-nitrophenyl)-5- (2,4-disulfophenyl)-2H-tetrazolium] according to the manufacturer's instructions (Dojindo Molecular Technologies). Optical densities were measured at 450 nm using a MultiskanTM Go Microplate plate reader (Thermo Fisher Scientific, Waltham, MA, USA).

Apoptosis was evaluated by means of the annexin V/7AAD staining assay kit, according to the instructions provided by the manufacturer (R&D Systems Inc), and analyzed in a FACSCanto II flow cytometer (Becton Dickinson).

### Mitochondrial damage and protein expression analysis

The controls that are untreated and the cells (106 cells per assay) treated with WIN-55,212-2 (50 µM) for 15 and 30 minutes at 37°C were stained with 5 µM MitoSOX probe [Molecular Probes (Invitrogen)] to detect the mitochondrial superoxide. The MitoSOX signal was detected by flow cytometry.

The detailed methodology for analyzing mitochondrial membrane potential (Δψm), Western Blot analysis, and immunocytofluorescence analysis has been described elsewhere 29

### Quantification of ceramides

Cell lines HL60, KG-1a, and U937 (20x106 cells per condition) were treated with WIN-55,212-2 (50 µM) throughout different periods of time and the lipids were extracted with a 2:1 chloroform/methanol solution.

The culture cell lysate chloroform extracts were treated with 20 µl of internal standard (a solution of 826 µg of ceramide C17:0 in 25 ml of methanol) and were dried under a constant nitrogen stream at room temperature. The ceramides were reconstituted with 350 µl of an equimolecular mixture of methanol/formic acid (99:1, which contained 5 mM ammonium formate) and 2-propanol/formic acid (99:1).

Reconstituted samples (20 µl) were analyzed using a liquid chromatography system from Agilent (1200 series) featuring a binary pump (G1312A) connected to an API 2000 triple quadrupole mass spectrometer (Applied Biosystems) using an electrospray ionization interface in positive ionization mode (ESI +). The ceramides were separated in a Zorbax Eclipse XDB - C18 column (150 x 4.6 mm, 5 µm) from Agilent. The working buffer was a 70:30 mixture of methanol/formic acid (99:1, which contained 5 mM ammonium formate) and 2-propanol/formic acid (99:1). The mobile phase was supplied at 0.5 ml/min in isocratic mode. This method provided the effective separation of the eight ceramides analyzed and the IS. The mass spectrometry was acquired by means of multiple reaction monitoring (MRM). The nebulizing gas (synthetic air), the gas of the curtain (nitrogen), and the gas of the heater (synthetic air) were set at 45, 25, and 45 (arbitrary units), respectively. The collision gas (nitrogen) was set at 3 (arbitrary units). The temperature of the gas of the heater was set at 500°C and the electrospray capillary voltage at 5.5 kV.

The eight ceramides studied were quantified using calibration curves in which ceramide 17:0 was used as an internal standard. The ceramide content was directly proportional to the ceramide/internal standard ratio (r> 0.99, p <0.01). The relative standard deviations (RSD) were <10%.

### Murine model of AML

The experiments with animals described in this study were carried out according to the accepted standards of animal care and Spanish regulations for the wellbeing of animals used in experimental neoplasm studies, and the study was approved by the institutional committee on animal care.

The NOD/scid/IL-2R gammae null (NSG) mice were acquired from Charles River Laboratories International (L'Arbresle, France) and received food and water *ad libitum,* under specific pathogen-free conditions. When the mice were 8-12 weeks old, AML was induced by intravenous inoculation of the HL60 cell line, and the mice were monitored to confirm progression of the disease by means of studying weight loss and detecting human CD45 + cells in bone marrow BM aspirates and flow cytometry analysis. Once the presence of leukemia cells was confirmed, treatment with a vehicle, WIN-55 212 cannabinoid was administered at a dose of 5 mg/kg/day or cytarabine (ARA-C) at 50 mg/kg for 5 days.

The effect of the cannabinoid on normal hematopoiesis was also tested by means of the treatment of healthy BALB/c mice with WIN-55,212-2 at a dose of 5 mg/kg/day for 7 and 28 days. The bone marrow and the peripheral blood population were analyzed by means of flow cytometry and blood counts.

### Statistical analysis

For all the statistical analyses, SPSS software version 15.0 (Statistical Package for the Social Sciences, SPSS, Chicago, IL, USA) was used and the statistical significance was defined as P ≤ 0.05. The error bars represent the standard error of the mean (SEM). The data was analyzed using the Student's T-test.

### Results

### WIN-55, 212-2 and the PGN cannabinoid family are cytotoxic for leukemia cell lines

Now it will be analyzed whether the exposure to cannabinoids had any effect on the viability of the tumor cells *in vitro.* To that end, the human AML U937, HL60, KG-1a, NB-4, MOLM-13, and KG-1 cell lines were cultured in serum-free medium and exposed to various concentrations of WIN-55,212-2 (range of 100 nM to 50 µM) for 18 hours, and cell viability was measured by means of WST-1 assays (Table 1). The three most sensitive cell lines are U937, HL60, and KG-1a.

**Table 3. Sensitivity of leukemia cell lines to the response with WIN-55**

| | | WIN-55 response | |
|---|---|---|---|
| *Cell line* | *Cell type* | *% viable cells at 50 µM* | *IC50 (µM)* |
| U937 | histiocytic lymphoma | 17.2 | 16.0 |
| HL60 | acute promyelocytic leukemia | 28.4 | 19.4 |
| KG-1a | acute myelogenous leukemia | 42.1 | 22.0 |
| NB-4 | acute promyelocytic leukemia | 49.1 | 25.1 |
| MOLM-13 | acute myelogenous leukemia | 56.6 | 25.9 |
| KG-1 | acute myelogenous leukemia | 57.4 | 33.2 |

The effect of WIN-55,212-2 and the PGN cannabinoid family on these three cell lines at 18, 48, and 72 hours was then analyzed by WST-1 assays and flow cytometry (supporting information of Figure 8, Figure 15) and primary cells from healthy donors (normal T lymphocytes, B lymphocytes, and HSC) at 18 hours. As shown in Figure 8A, the results showed that exposure to all cannabinoids at concentrations of 10 µM or higher led to a significant reduction in the AML cell line viability in a concentration-dependent manner. In contrast, the viability of the normal cell subpopulations from healthy donors was not affected. It is important to point out that it was verified by means of flow cytometry analysis that the reduction in AML cell growth induced by cannabinoids was in fact due to apoptotic cell death. Figure 8B shows the results of the Annexin V/7AAD bivariate analysis of exponential AML cell growth. The results indicated that the cannabinoids reduced cell viability by increasing apoptosis, as is evidenced by a significant increase in the number of Annexin V+/7AAD+ cells (Figure 8B), whereas the number of normal annexin V-/7AAD-(live) cell subpopulations of healthy donors remained constant (Figure 8C).

The effects of the selective CB2 antagonists on cytotoxicity induced by cannabinoids were finally examined. The HL60 and KG1-a cell lines were exposed to WIN-55,212-2 (10 µM) in the presence or absence of CB2 antagonist PGN-8, -37 and -70, and cell viability was determined by means of the WST-1 assay 18 hours later (Figure 9). The results showed that treatment with all the selective CB2 antagonists was capable of significantly inhibiting cannabinoid-induced apoptosis. Together, these results suggested that the exposure of AML cell lines to cannabinoids ≥10 µM *in vitro* led to the death of CB2-dependent cells by induction of apoptosis.

### WIN-55.212-2 induces the cleavage of caspases, which is blocked using a pan-caspase inhibitor.

Activation of the caspase cascade is commonly associated with the induction of the apoptosis. Therefore, to elucidate the role of caspases in cannabinoid-induced apoptosis, the caspase activation pattern was examined after treatment with WIN-55,212-2. For this purpose, the HL60 cells were treated with this cannabinoid at a concentration of 50 µM or a vehicle for 2, 6, 18, and 24 hours. The cells were then collected and the expression of the various caspases was determined by means of Western blot analysis (Figure 10A). The results demonstrate that exposure to WIN-55,212 2 led to the activation of various caspases. More specifically, the cleavage of effector-type caspase-3 and poly(ADP-ribose) polymerase (PARP) and the reduction in procaspase-2, -8 and -9 were observed. This indicates that WIN-55,212-2 activates intrinsic and extrinsic apoptotic pathways.

To further investigate the importance of caspases in the anti-leukemia action of cannabinoids, the capacity of the pan-caspase inhibitor Z-VAD(OMe)-FMK to rescue cells from cell death induced by cannabinoids was evaluated. The cells were preincubated for 60 minutes with the pan-caspase inhibitor, then WIN-55,212-2 was added and the incubation continued for 18 hours. As shown in Figure 10B, the pro-apoptotic effect of cannabinoids on AML cells was abolished following co-culture with pan-caspase inhibitors.

### WIN-55,212-2 induces early mitochondrial damage and ER stress

Since the cleavage of caspase 9 is a very early event in cannabinoid-induced apoptosis, and this change indicates activation of the intrinsic apoptosis pathway, the effect of WIN-55,212-2 on mitochondria, which are critically involved in triggering apoptosis through this pathway, was studied. For the purpose of further investigating the participation of the mitochondrial pathway in cannabinoid-induced apoptosis, the effect of WIN-55,212-2 treatment on Δψm in the HL60 cell line was studied.

Exposure of the HL60 cells to 50 µM for 15 or 30 minutes led to a significant reduction in the mitochondrial membrane potential, as shown in Figure 11A.

Furthermore, ROS have been associated with the activation of the intrinsic apoptotic pathway. In parallel, the production of ROS was studied using the fluorochrome MitoSox targeting mitochondria in AML and HSC cells. The results showed that the exposure of HL60 cells to cannabinoids led to a significant increase in ROS production levels at 15 minutes or more. In contrast, this experiment was reproduced in hematopoietic stem cells, but ROS levels remained unchanged (Figure 11B].

Finally, ER stress in HL60 cells was studied by Western blot analysis. The cannabinoid increased the expression of crucial factors in the response of the unfolded protein (EPU) to ER stress, such as p-IRE1, p-PERK, and CHOP (Figure 11C).

### The accumulation of ceramides is involved in cannabinoid-induced apoptosis

Given that it has been reported that the new synthesized ceramide is involved in cannabinoid-induced apoptosis, it was investigated if the cannabinoid derivatives also act through a similar pathway in leukemia cells. Experiments were conducted with selective ceramide synthesis inhibitors, such as myriocin and fumonisin B1 (inhibitors of serine palmitoyltransferase (SPT) and ceramide synthase, respectively) (Figure 12A).

The pharmacological blockade of ceramide synthesis with fumonisin B1 only partially prevented the decrease in the viability of HL60 cells after exposure to cannabinoids, evaluated by means of WST-1 assays at 18 hours (Figure 12B). In contrast, it prevented the fragmentation of PARP and the cleavage of Casp 3 as it was evaluated by means of Western blot analysis when HL60 cells were treated with myriocin (Figure 12C).

Furthermore, significant differences in the amounts of certain subtypes of ceramides in untreated leukemia cells compared to those that are treated were observed by means of immunohistochemistry and HPLC/MS-MS in HL60 cells (Figure 12D and 12E), U937 cells (Figure 9) and KG-1a cells (supporting information, Figure 9). This data confirmed that ceramide plays a crucial role in cannabinoid-induced apoptosis in AML cells.

Oddly enough, ceramides 16:0, 18:0, and 18:1 followed a constant growth pattern up to 24 hours, whereas the ceramides having a longer chain reached their maximum level at 6 hours and then returned to their baseline levels.

### Signaling pathways targeted by cannabinoids in AML cells.

It has been demonstrated that AKT, ERK, JNK, and p38 MAPK regulation plays an important role in the survival or induction of apoptosis in a series of cell types. Therefore, it was examined if exposure to WIN-55,212-2 had any effect on the levels of the phosphorylated forms of these signaling molecules. For this purpose, the HL60 cells were exposed to a vehicle or to 50 µM of WIN-55,212-2 for 2, 6, 18, and 24 hours. The cells were then marked with antibodies specific for p-AKT, p-ERK, p-JNK, and p-p38 MAPK and studied by means of Western blot analysis (Figure 13A).

The Western blot assays demonstrated that treatment with cannabinoids slightly over-regulates p-JNK and p-Erk1/2, whereas it slightly under-regulates p-p38-MAPK and p-AKT over time.

Expression of the pro-apoptotic regulator Bax is also studied by immunocytofluorescence analysis. It has been reported that this protein is involved in ceramide-induced apoptosis. Figure 13B showed a considerable increase in Bax when the HL60 cells were treated with WIN-55,212-2.

### Antitumor effect of the cannabinoids on murine models of AML in vivo

For the purpose of evaluating the effect of cannabinoids *in vivo* on normal HSC, BALB/c mice were treated with 5 mg/kg/day of WIN-55,212-2 for 7 and 28 days, and the different subpopulations of HSC identified by flow cytometry were evaluated. It has been confirmed that the cannabinoids do not affect the viability of the different hematopoietic progenitor populations and increase the number of cells under some conditions (Figure 14A).

Furthermore, peripheral blood populations were analyzed by blood count studies and an increase in blood platelet count was observed in treated mice (Figure 14B).

Finally, in order to study the effects of cannabinoid on the growth of human AML cells *in vivo,* HL60 cell xenograft models were used. For this purpose, NSG mice were treated with 5 mg/kg/day of WIN-55,212-2, 50 mg/kg of ARA-C for 5 days, or placebo once bone marrow infiltration with leukemia cells, evaluated by flow cytometry, is confirmed. Figure 14C provides an example of flow cytometry analysis for the human cell contained in mouse bone marrow. Treatment with WIN-55.212-2 induced a vast reduction in the number of HL60 cells in bone marrow. Furthermore, a significant increase in survival between the mice treated with WIN-55 and cannabinoid was observed compared with the control group and the group treated with ARA-C (Figure 14D).

## Claims

1. Use of a compound of general formula (I): 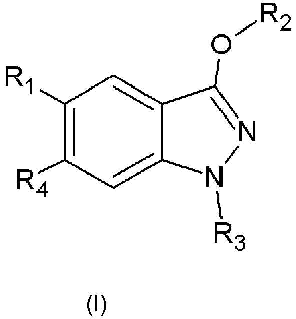 where
R1 and R4 are members of the group consisting of hydrogen, halogen, nitro, or amino;
R2 is a member of the group consisting of propyl, butyl, pentyl, cyclohexylmethyl, phenethyl, naphthylmethyl, heterocycloalkyl, primary, secondary or tertiary amine, or substituted benzyl, wherein the phenyl group may contain 1 or 2 substituents of the group consisting of alkyl, hydroxy, methoxy, nitro, amino, or halogen;
R3 is a member of the group consisting of methyl, ethyl, propyl, pentyl, cycloalkylmethyl, cycloalkylethyl, dialkylaminoethyl, heterocycloalkylethyl, cycloalkylcarbonyl (carbonyl group attached to cycloalkyl), heteroarylcarbonyl (carbonyl group attached to heteroaryl), optionally substituted arylcarbonyl (carbonyl group attached to aryl), or optionally substituted aralkylcarbonyl (carbonyl group attached to aralkyl);
or any of the pharmaceutically acceptable salts, esters, tautomers, solvates, and hydrates thereof, or any of the combinations thereof, in the production of a medicinal product for the prevention, relief, improvement, and/or treatment of cancer.

2. Use of the compound according to claim 1, wherein the cancer is a hematological cancer.

3. Use of the compound according to any of claims 1 to 2, wherein the hematological cancer is selected from acute myeloid leukemia or monoclonal gammopathy.

4. Use of the compound according to any of claims 1 to 3, where:
R1 is a member of the group consisting of hydrogen or amino;
R2 is a member of the group consisting of 4-methoxybenzyl, 1-naphthylmethyl, 2-naphthylmethyl, heterocycloalkyl, diisopropylamino, dimethylamino, diethylamino, piperidinyl, morpholinyl, or pyrrodinyl;
R3 is a member of the group consisting of piperidinoethyl, morpholinoethyl, pyrrolidinylethyl, diisopropylaminoethyl, optionally substituted aryl, optionally substituted aralkyl, 2-thienyl, or 4-chloro-3-pyridyl;
R4 is hydrogen.

5. Use of the compound according to any of claims 1 to 4, where:
R1 is a member of the group consisting of hydrogen or amino;
R2 is a member of the group consisting of 4-methoxybenzyl, 1-naphthylmethyl, or 2-naphthylmethyl;
R3 is a member of the group consisting of piperidinoethyl, morpholinoethyl, pyrrolidinylethyl, or diisopropylaminoethyl;
R4 is hydrogen.

6. Use of a compound according to any of claims 1 to 5, wherein the compound is selected from the list consisting of: 3-(2-naphthylmethoxy)-1-(2-piperidinoethyl)indazole, 3-(1-naphthylmethoxy)-1-(2-piperidinoethyl)indazole, 1-(cyclohexylmethyl)-3-(cyclohexylmethoxy)indazole, 1-methyl-3-(2-naphthylmethoxy)indazole, 1-(2-cyclohexylethyl)-3-(2-naphthylmethoxy)indazole, 1-methyl-3-(3,4-dimethylbenzyloxy)indazole, 3-(2-naphthylmethoxy)-5-nitro-1-(2-piperidinoethyl)indazole, 3-(2-naphthylmethoxy)-5-nitro-1-pentylindazole, 1-methyl-3-(2-naphthylmethoxy)-5-nitroindazole, 1-methyl-5-nitro-3-(phenethoxy)indazole, 5-nitro-1-pentyl-3-(pentyloxy)indazole, 3-(3,4-dimethylbenzyloxy)-1-(2-morpholinoethyl)-5-nitroindazole, 1-methyl-3-(1-naphthylmethoxy)-5-nitroindazole, 1-(2-morpholinoethyl)-3-(2-naphthylmethoxy)-5-nitroindazole, 3-(3,4-dimethylbenzyloxy)-1-methyl-5-nitroindazole, 3-(1-naphthylmethoxy)-1-(2-(1-pyrrolidinyl)ethyl)-5-nitroindazole, 1-(cyclohexylmethyl)-3-(3,4-dimethylbenzyloxy)-5-nitroindazole, 5-bromo-3-(2-naphthylmethoxy)-1-(2-piperidinoethyl)indazole, 1-(2-(diisopropylamino)ethyl)-3-(4-methoxybenzyloxy)indazole, 5-amino-3-(2-naphthylmethoxy)-1-(2-piperidinoethyl)indazole, 3-(4-methoxybenzyloxy)-5-nitro-1-pentylindazole, 3-(2-naphthylmethoxy)-5-nitro-1-propylindazole, or any of the pharmaceutically acceptable salts, esters, tautomers, solvates, and hydrates thereof, or any of the combinations thereof.

7. Use of the compound according to any of claims 1 to 6, wherein the monoclonal gammopathy is selected from multiple myeloma, plasma cell leukemia, Waldenstrom's macroglobulinemia, amyloidosis, or any of the combinations thereof.

8. Use of the compound according to any of claims 1 to 7, wherein the monoclonal gammopathy is multiple myeloma.

9. Use of a composition comprising a compound defined in any of claims 1 to 8, or any of the pharmaceutically acceptable salts, esters, tautomers, solvates, and hydrates thereof, or any of the combinations thereof, in the production of a medicinal product for the prevention, relief, improvement, and/or treatment of cancer.

10. Use of a composition according to claim 9, wherein the cancer is a hematological cancer.

11. Use of a composition according to any of claims 9 to 10, wherein the cancer is selected from acute myeloid leukemia or monoclonal gammopathy.

12. The use of a composition according to any of claims 9 to 11, wherein the composition further comprises one or more pharmaceutically acceptable excipients.

13. Use of a composition according to any of claims 9 to 12, wherein the composition further comprises another active ingredient.

14. Use of a composition according to the preceding claim, wherein the other active ingredient is selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof.

15. Use of a composition according to any of claims 13 to 14, wherein the other active ingredient is dexamethasone.

16. Use of a composition according to any of claims 13 to 14, wherein the other active ingredient is melphalan.

17. Use of a composition according to claims 9 to 16, wherein the monoclonal gammopathy is selected from multiple myeloma, plasma cell leukemia, Waldenstrom's macroglobulinemia, amyloidosis, or any of the combinations thereof.

18. Use of a composition according to claims 9 to 17, wherein the monoclonal gammopathy is multiple myeloma.

19. A combined preparation comprising or consisting of:
a) a compound according to any of claims 1 to 8, or a composition according to any of claims 9 to 18, and
b) an active ingredient selected from the list consisting of prednisone, dexamethasone, doxorubicin, plerixafor, cyclophosphamide, granulocyte colony-stimulating factor, melphalan, thalidomide, lenalidomide, pomalidomide, bortezomib, carfilzomib, ixazomib, daratumumab, isatuximab, MOR202, elotuzumab, autologous stem cells (sASCT), allogeneic stem cells, or any of the combinations thereof.

20. The combined preparation according to claim 19, wherein the active ingredient of (b) is dexamethasone.

21. The combined preparation according to claim 19, wherein the active ingredient of (b) is melphalan.

22. Use of a combined preparation according to any of claims 19 to 21, wherein components (a) and (b) are administered simultaneously, separately, or sequentially for the prevention, relief, improvement, and/or treatment of cancer.

23. Use of a combined preparation according to claim 22, wherein the cancer is a hematological cancer.

24. Use of a combined preparation according to any of claims 22 to 23, wherein the cancer is selected from acute myeloid leukemia or monoclonal gammopathy.

25. Use of a combined preparation according to claim 24, wherein the monoclonal gammopathy is selected from multiple myeloma, plasma cell leukemia, Waldenstrom's macroglobulinemia, amyloidosis, or any of the combinations thereof.

26. Use of a combined preparation according to any of claims 23 to 25, wherein the monoclonal gammopathy is multiple myeloma.
